(19) Europäisches Patentamt
European Patent Office
Office européen des brevets

(11) EP 0 852 225 B1

(12) **EUROPEAN PATENT SPECIFICATION**

(45) Date of publication and mention of the grant of the patent:
**30.10.2002 Bulletin 2002/44**

(21) Application number: **97928513.7**

(22) Date of filing: **27.06.1997**

(51) Int Cl.[7]: **C07D 211/34**, C07D 401/12, C07D 417/12, A61K 31/445, A61K 31/495, A61K 31/55, C07D 211/46

(86) International application number:
**PCT/JP97/02252**

(87) International publication number:
**WO 97/049682 (31.12.1997 Gazette 1997/57)**

(54) **FIBRINOGEN RECEPTOR ANTAGONISTS AND MEDICINAL PREPARATIONS CONTAINING THE SAME AS THE ACTIVE INGREDIENT**

FIBRINOGEN- REZEPTOR- ANTAGONISTEN UND MEDIZINISCHE ZUBEREITUNGEN DIE DIESE ALS AKTIVEN BESTANDTEIL ENTHALTEN

ANTAGONISTES DE RECEPTEUR DE FIBRINOGENE, ET PREPARATION MEDICINALE CONTENANT CES ANTAGONISTES EN TANT QU'INGREDIENT ACTIF

(84) Designated Contracting States:
**AT BE CH DE ES FR GB IT LI NL SE**

(30) Priority: **27.06.1996 JP 16798296**

(43) Date of publication of application:
**08.07.1998 Bulletin 1998/28**

(73) Proprietor: **NIPPON STEEL CORPORATION**
**Tokyo 100-71 (JP)**

(72) Inventors:
- **HAYASHI, Yoshio, Nippon Steel Corporation Kawasaki-shi, Kanagawa 211 (JP)**
- **HARADA, Takeo, Nippon Steel Corporation Kawasaki-shi, Kanagawa 211 1 (JP)**
- **TACHIKI, Akira, Nippon Steel Corporation Kawasaki-shi, Kanagawa 211 11 (JP)**
- **KATADA, Jun, Nippon Steel Corporation Kawasaki-shi, Kanagawa 211 (JP)**

(74) Representative: **VOSSIUS & PARTNER**
**Postfach 86 07 67**
**81634 München (DE)**

(56) References cited:

| | |
|---|---|
| **EP-A- 0 445 796** | **WO-A-92/15607** |
| **WO-A-96/20172** | **JP-A- 4 217 652** |

- **HARADA T ET AL: "Development of the new potent non-peptide GpIIb/IIIa antagonist NSL-95301 by utilizing combinatorial technique" BIOORG. MED. CHEM. LETT. (BMCLE8,0960894X);97; VOL.7 (2); PP.209-212, LIFE SCIENCE RESEARCH CENTER NIPPON STEEL CORPORATION;ADVANCED TECHNOLOGY RESEARCH LABORATORIES; KAWASAKI; 211; JAPAN (JP), XP000618322**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

TECHNICAL FIELD

The present invention relates to novel compounds having a platelet aggregation-inhibiting action and pharmaceutical preparations comprising the compounds as active ingredient which can be used as agents for inhibiting platelet aggregation, blood coagulation during extracorporeal circulation and reocclusion of coronary arteries.

BACKGROUND ART

Platelets in blood play such an important role in hemostasis that they adhere to the surface of an injured blood vessel to prevent bleeding therefrom. However, under diseased conditions, platelet aggregation is known to be primarily responsible for the formation of thrombi, which causes the obstruction of blood vessels. This obstruction prevents the adequate supply of oxygen and nutrients to tissues and organs and thereby causes ischemic diseases in circulatory organs such as myocardial infarction and cerebral infarction. At present, the high mortality from these ischemic diseases has become a serious social problem.
When medical treatments involving the extracorporeal circulation of blood, as exemplified by the use of artificial hearts and lungs during surgical operations and renal dialysis for patients with renal failure, are conducted, blood coagulation is sometimes caused during the extracorporeal circulation of blood by the activation and aggregation of platelets, which is a great obstacle to the performance of such medical treatments.
It is suggested that platelet aggregation partakes in acute reocclusion after pericutaneous transluminal coronary angioplasty (PTCA) as applied to thrombi in coronary arteries in patients with cardiac infarction.
Hence, prevention of thrombus formation, blood coagulation and reocclusion of coronary arteries after the operation by inhibiting platelet aggregation is very important for the purpose of preventing or treating ischemic diseases or performing medical treatments through extracorporeal circulation in a safe manner. It has become known in recent year that platelet aggregation also plays an important role in the progress of arterial sclerosis.
The process of platelet aggregation consists of two stages, i.e., the activation of platelets and a subsequent aggregation mediated by cross-linking protein "fibrinogen" in plasma. Almost all of the conventional platelet aggregation-inhibiting agents target the first activation process. These agents include cyclooxygenase inhibitor aspirin, adenylate cyclase activator ticlopidine, phosphodiesterase inhibitor dipyridamole and the like. These compounds are not satisfactory in the specificity of action and the aggregation-inhibiting activity. Therefore, there is a need for the development of pharmaceutical agents having a more specific and potent action.
As regards the aggregation process mediated by fibrinogen, it has been known that fibrinogen is associated with platelets by a very highly specific binding to glycoprotein "gpIIbIIIa" which is a fibrinogen receptor on the surface of platelet membranes. Inhibition of such platelet-specific binding will lead to the development of a highly specific drug. Inhibiting the binding of fibrinogen to platelets will also contribute to the creation of a potent and highly specific platelet aggregation-inhibiting agent because even activated platelets cannot aggregate if the fibrinogen-mediated aggregation process is inhibited.
In a study from the viewpoint of molecular biology, Andrieux et al. found that the binding of fibrinogen to a fibrinogen receptor is primarily dependent on an amino acid sequence in the fibrinogen molecule, that is, arginine-glycine-aspartic acid-phenylalanine (RGDF) (Andrieux et al., J. Biol. Chem., vol. 264, pp. 9258-9265, 1989).
An attempt was made to synthesize this partial peptide and its analogues and use them as fibrinogen receptor antagonists. Japanese Unexamined Patent Publication (hereinafter referred to as "KOKAI") Nos. Hei 1-190699 and Hei 2-62892, EPO 422937 A1 and United States Patent (hereinafter referred to as USP") No. 4952562 disclose tetrapeptide derivatives containing the RGD peptide. KOKAI No. Sho 63-215696 discloses derivatives consisting of peptides. KOKAI Nos. Hei 3-118331 and Hei 2-62892 and WO91/01331 disclose derivatives having the cyclic structure of the RGD peptide.
The RGD peptide is characterized in that it is digested in vivo with a protease to amino acids which are safe and useful to the organism. Based on the finding of such a characteristic property, the inventors thought that for uses which did not require the sustained action of drugs, such as extracorporeal circulation and surgical operation, the creation of highly active peptide compounds having structures as similar to native peptides as possible was important for the development of platelet aggregation-inhibiting agents having few or no side effects. As a result of the intensive studies the inventors conducted, they developed novel peptides as described in KOKAI Nos. Hei 4-23864, Hei 5-203962, Hei 6-139107 and Hei 6-235745.
There are also reports on so-called peptidomimetics in which peptide structures containing more or less native amino acids were further denvatized and/or modified as described in KOKAI No. Hei 3-248808, WO93/16697, EP0503548, EP0502536, WO93/08181, WO93/08174, WO93/07867, WO94/08577, EP0445796 and EP0505868.
In general, compounds having chemical structures stable in vivo are required to develop drugs that need a

sustained action. In the case of oral drugs, the stability and bioavailability of compounds in digestive tracts must also be taken into consideration. Peptides are generally unsuitable for such drugs of long lasting action because of their low stability.

BIORG.MED.CHEM.LETTERS, vol 7(2), pp209-212 discloses the preparation of various modified RGD peptides in which an N-terminal region, a spacer and a C-terminal region were divided and each independently modified. It also shows platelet-aggregation inhibitory activity of these peptides. WO92/15607 discloses platelet-aggregation-inhibition-active peptidemimetics having an amidine group. WO96/20172 discloses compounds in which one hydrogen atom bound to the terminal nitrogen atom in an amidine group is substituted.

EP445796 discloses acetic acid derivatives having β-alanine residues which have a platelet thrombus formation-inhibiting action. The inventors have been developed independently acetic acid derivatives having a β-alanine residue or mono-substituted β-alanine residue. However, these compounds do not have a sufficiently high biological activity to be used for practical purposes and the development of compounds having a higher biological activity has therefore been desired.

An object of the present invention is to provide novel compounds that antagonize the action of fibrinogen receptors and which have a high platelet aggregation-inhibiting activity, in vivo stability and high bioavailability, as well as novel pharmaceutical preparations comprising the compounds as active ingredient.

DISCLOSURE OF THE INVENTION

As a result of the various studies made to solve the aforementioned problems, the inventors have found that β-amino acid derivatives having two lower alkyl groups at the α position show a high platelet aggregation-inhibiting activity and a high blood coagulation-inhibiting activity and that the physiological activities of the β-amino acid derivatives can be increased by modification of the derivatives at the β position, Based on these findings, the inventors have accomplished the present invention.

The present invention provides compounds of the following general formula (I) and pharmaceutically acceptable salts thereof:

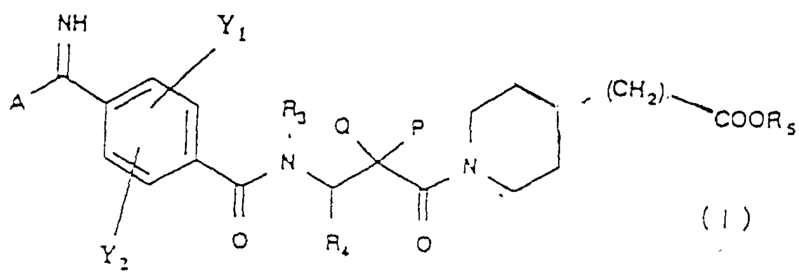

( I )

wherein A is a tertiary amine;

$R_3$ is hydrogen, a lower alkyl, lower alkenyl, lower alkynyl, ar(lower)alkyl or aryl;

$R_4$ is hydrogen, a lower alkyl, lower alkenyl, lower alkynyl, hydroxy(lower)alkyl, nitrooxy(lower)alkyl, nitrosooxy(lower) alkyl, amino(lower)alkyl or heterocycle-substituted lower alkyl; an ar(lower)alkyl, ar(lower)alkenyl or ar(lower)alkynyl the aryl moiety of which may have a lower alkyl, halogen, nitro, amino, carboxyl, hydroxy(lower)alkyl, hydroxyl or protected hydroxyl; an aryl or heterocyclic group which may have a lower alkyl, halogen, nitro, amino, carboxyl, hydroxy (lower)alkyl, hydroxyl or protected hydroxyl; a cycloalkyl with a 3-8 membered ring the ring moiety of which may have a lower alkyl, halogen, nitro, amino, carboxyl, hydroxy(lower)alkyl, hydroxyl or protected hydroxyl, or a lower alkyl, lower alkynyl or lower alkenyl which are substituted with the cycloalkyl; or a lower alkyloxy;

P and Q are each

$R_5$ is hydrogen or a physiologically cleavable carboxyl-protecting group;

$Y_1$ and $Y_2$ are each independently hydrogen, a lower alkyl, halogen, hydroxyl, lower alkoxy, lower acyloxy, acyl, caboxyl, lower alkoxycarbonyl, nitro or trifluoromethyl; and

In general formulation (I), A may be a cyclic tertiary amine including, for example, a tertiary amine represented by the following general formula (II), (III) or (IV):

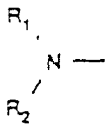, 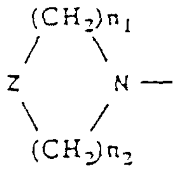, or 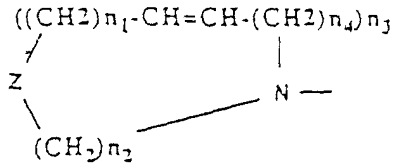

(II) (III) (IV)

wherein $R_1$ and $R_2$ are each independently a lower alkyl, lower alkenyl, lower alkynyl, ar(lower) alkyl or aryl;
Z is oxygen, sulfur,

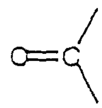, 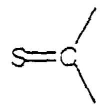;

carbon having hydrogen, a lower alkyl, lower alkenyl, lower alkynyl, ar(lower)alkyl, hydroxyl or substituted hydroxyl, amino or substituted amino, nitro, nitrooxy, nitrosooxy, halogen, thiol or substituted thiol, or aryl as its side chain; or nitrogen having hydrogen, a lower alkyl, lower alkenyl, lower alkynyl, ar(lower)alkyl, hydroxyl or substituted hydroxyl, amino or substituted amino, nitro, nitroso or aryl as its side chain;
$n_1$, $n_2$ and $n_4$ are each independently an integer of 0 to 6 and $n_3$ is an integer of 1 to 3.

In general formula (I), P and Q are both methyl.

The present invention further provides pharmaceutical preparations comprising the aforementioned compounds or pharmaceutically acceptable salts thereof as active ingredient which can be used as agents for inhibiting platelet aggregation, blood coagulation during extracorporeal circulation or reocclusion of coronary arteries.

## BRIEF DESCRIPTION OF THE DRAWINGS

Figure 1 shows time course of the concentration of the compound of of Example 3 in blood after oral administration.

## BEST MODE FOR CARRYING OUT THE INVENTION

In the compounds of the present invention, the α and β positions mean the 2 and 3 positions, respectively, of a β-alanine residue, that is, 3-aminopropionic acid residue.

Suitable examples and explanation of various definitions involved in the scope of the present Invention will be described below.

Unless otherwise indicated, the term "lower" means groups having 1-10 carbon atoms, preferably 1-6 carbon atoms, and more preferably 1-3 carbon atoms.

In general formula (I), the tertiary amine represented by A may be a cyclic or non-cyclic tertiary amine, for example, a tertiary amine represented by the following general formula (II), (III) or (IV):

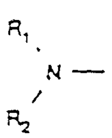, 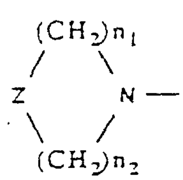, or 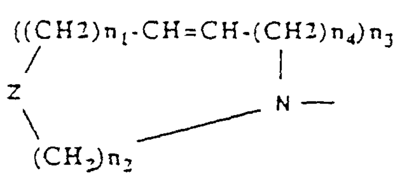

(II) (III) (IV)

wherein $R_1$ and $R_2$ are each independently a lower alkyl, lower alkenyl, lower alkynyl, ar(lower) alkyl or aryl which may have a hydroxyl, amino, nitro, nitrooxy, nitrosooxy, halogen or alkoxy such as methoxy on its side chain;
Z is oxygen, sulfur,

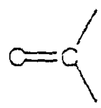 , 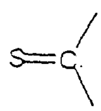 ;

carbon having hydrogen, a lower alkyl, lower alkenyl, lower alkynyl, ar(lower)alkyl, hydroxyl or substituted hydroxyl, amino or substituted amino, nitro, nitrooxy, nitrosooxy, halogen, thiol or substituted thiol, or aryl on its side chain; or nitrogen having hydrogen, a lower alkyl, lower alkenyl, lower alkynyl, ar(lower)alkyl, hydroxyl or substituted hydroxyl, amino or substituted amino, nitro, nitroso or aryl on its side chain;

$n_1$, $n_2$ and $n_4$ are each independently an integer of 0 to 6 and $n_3$ is an integer of 1 to 3.

Preferred examples of the "lower alkyl" for $R_1$ and $R_2$ may include C1-10 straight-chain, branched-chain or cyclic alkyls such as methyl, ethyl, propyl, isopropyl, cyclopropyl, butyl, isobutyl, tert-butyl, pentyl, isopentyl, cyclopentyl, isohexyl, cyclohexyl, heptyl, 5-methylhexyl, cycloheptyl, octyl, 6-methylheptyl, nonyl, 7-methyloctyl, decyl and 8-methylnonyl. Considering steric hindrance, C1-4 alkyls are preferred.

Preferred examples of the "lower alkenyl" for $R_1$ and $R_2$ include C2-10 straight- or branched-chain alkenyls such as vinyl and propenyl. Considering steric hindrance, C2-4 alkenyls are preferred.

Preferred examples of the "lower alkynyl" for $R_1$ and $R_2$ include C2-10 straight- or branched-chain alkynyls such as ethynyl, propynyl and butynyl. Considering steric hindrance, C2-4 alkynyls are preferred.

Preferred examples of the "ar(lower) alkyl" for $R_1$ and $R_2$ include phenylalkyls such as benzyl and phenethyl; and those alkyls having an aromatic heterocycle such as pyridyl and furyl. In these cases, the aryl moiety such as phenyl may have a lower alkyl, hydroxy(lower)alkyl or hydroxyl, where the hydroxyl includes a protected hydroxyl. The lower alkyl in the ar(lower)alkyl and hydroxy(lower)alkyl is preferably a C1-3 alkyl having small steric hindrance. Preferred examples of the protective groups in the "protected hydroxyl" include ar(lower)alkyls such as benzyl, phenethyl and trityl; lower alkyls such as methyl, ethyl, propyl, isopropyl and tert-butyl; acyls such as acetyl and benzoyl; nitro, nitroso, tetrahydropyranyl and methoxymethyl. Preferred examples of the ar(lower)alkyl include phenyl, benzyl, 4-hydroxybenzyl and 4-hydroxymethylbenzyl.

Preferred examples of the "aryl" for $R_1$ and $R_2$ include phenyl; aromatic heterocycles such as pyridyl, furyl, thiophene, oxazolyl and thiazolyl; and condensed polycyclic hydrocarbons such as naphthyl and anthranyl. In these cases, the aromatic ring may be substituted with at least of the substituents consisting of a lower alkyl, hydroxy(lower) alkyl, hydroxyl and protected hydroxyl. In general, the aromatic ring is suitable for the present invention because it takes on a planar structure and thereby has small steric hindrance and high hydrophobic property. Phenyl or pyridyl is a preferred aromatic ring. The lower alkyl per se or the lower alkyl in the hydroxy(lower)alkyl as a substituent on the aromatic ring is preferably a C1-3 alkyl having small steric hindrance, such as methyl, ethyl or propyl.

Preferred examples of the "lower alkyl" when Z is carbon or nitrogen include C1-10 straight-chain, branched-chain or cyclic alkyls such as methyl, ethyl, propyl, isopropyl, cyclopropyl, butyl, isobutyl, tert-butyl, pentyl, isopentyl, cyclopentyl, isohexyl, cyclohexyl, heptyl, 5-methylhexyl, cycloheptyl, octyl, 6-methylheptyl, nonyl, 7-methyloctyl, decyl and 8-methylnonyl. Considering steric hindrance, C1-6 alkyls are preferred.

Preferred examples of the "lower alkenyl" when Z is carbon or nitrogen include C2-10 straight- or branched-chain alkenyls such as vinyl and propenyl. Considering steric hindrance, C2-6 alkenyls are preferred.

Preferred examples of the "lower alkynyl" when Z is carbon or nitrogen include C2-10 straight- or branched-chain alkynyls such as ethynyl, propynyl and butynyl. Considering steric hindrance, C2-6 alkynyls are preferred.,

Preferred examples of the "ar(lower) alkyl" when Z is carbon or nitrogen include phenylalkyls such as benzyl and phenethyl; and those alkyls having an aromatic heterocycle such as pyridyl and furyl. In these cases, the aryl moiety such as phenyl may have a lower alkyl, halogen, hydroxy(lower)alkyl or hydroxyl, where the hydroxyl includes a protected hydroxyl. The lower alkyl in the ar(lower)alkyl and hydroxy(lower)alkyl is preferably a C1-3 alkyl having small steric hindrance. Preferred examples of the protective groups in the "protected hydroxyl" include ar(lower)alkyls such as benzyl, phenethyl and trityl; lower alkyls such as methyl, ethyl, propyl, isopropyl and tert-butyl; acyls such as acetyl and benzoyl; nitro, nitroso, tetrahydropyranyl and methoxymethyl. Preferred examples of the ar(lower)alkyl include phenyl, benzyl, 4-hydroxybenzyl and 4-hydroxymethylbenzyl.

Preferred examples of the "aryl" when Z is carbon or nitrogen include phenyl; aromatic heterocycles such as pyridyl, furyl, thiophene, oxazolyl and thiazolyl; or condensed polycyclic hydrocarbons such as naphthyl and anthranyl. In these cases, the aromatic ring may be substituted with at least one of the substituents consisting of a lower alkyl, halogen, hydroxy(lower)alkyl, hydroxyl and protected hydroxyl. In general, the aromatic ring is suitable for the present invention because it takes on a planar structure and thereby shows small steric hindrance and high hydrophobic property. Phenyl or pyridyl is a preferred aromatic ring. The lower alkyl per se or the lower alkyl in the hydroxy(lower)alkyl as a substituent on the aromatic ring is preferably a C1-3 alkyl having small steric hindrance, such as methyl, ethyl or propyl.

Preferred examples of the substituent of the "substituted hydroxyl" when Z is carbon or nitrogen include an ar(lower)alkyl such as benzyl, phenethyl and trityl; a lower alkyl such as methyl, ethyl, propyl, isopropyl and tert-butyl; an acyl such as acetyl and benzoyl; nitro, nitroso, tatrahydropyranyl and methoxymethyl.

Preferred examples of the substituent of the "substituted amino" when Z is carbon or nitrogen include an ar (lower)alkyl such as benzyl, phenethyl and trityl; a lower alkyl such as methyl, ethyl, propyl, isopropyl and tert-butyl; an acyl such as acetyl and benzoyl; a carbamate such as benzyloxycarbonyl and methoxycarbonyl; and nitroso.

Preferred examples of the substituent of the "substituted thiol" when Z is carbon include an ar(lower)alkyl such as benzyl, phenethyl and trityl; a lower alkyl such as methyl, ethyl, propyl, isopropyl and tert-butyl; and nitroso.

Preferred examples of the "halogen" when Z is carbon include chlorine, fluorine and bromine.

$n_1$, $n_2$ and $n_4$ are each independently an integer of 0 to 6, and preferably 1 to 3. $n_3$ is an integer of 1 to 3, and most preferably 1.

The lower alkyl" for $R_3$ includes C1-10 straight- and branched-chain alkyls and cyclic alkyls such as methyl, ethyl, propyl, isopropyl, cyclopropyl, butyl, isobutyl, tert-butyl, pentyl, isopentyl, cyclopentyl, hexyl, isohexyl, cyclohexyl, heptyl, 5-methylhexyl, cycloheptyl, octyl, 6-methylheptyl, nonyl, 7-methyloctyl, decyl and 8-methylnonyl. Considering steric hindrance, C1-6 alkyls are preferred. Straight-chain alkyls are preferred to branched-chain and cyclic ones.

The lower alkenyl" for $R_3$ includes C2-10 straight- and branched-chain alkenyls such as vinyl and propenyl. Considering steric hindrance, C2-6 alkenyls are preferred.

The lower alkynyl" for $R_3$ includes C2-10 straight- and branched-chain alkynyls such as ethynyl, propynyl and butynyl. Considering steric hindrance, C2-6 alkynyls are preferred.

The ar(lower)alkyl" for $R_3$ includes phenylalkyl such as benzyl and phenethyl. In this case, the aryl moiety such as phenyl may have a lower alkyl, hydroxy(lower)alkyl or hydroxyl, where the hydroxyl includes a protected hydroxyl. The lower alkyl in the ar(lower)alkyl and hydroxy(lower)alkyl is preferably a C1-3 alkyl having small steric hindrance. The protective groups in the protected hydroxyl" include ar(lower)alkyls such as benzyl, phenethyl and trityl, lower alkyls such as methyl, ethyl, propyl, isopropyl and tert-butyl, acyls such as acetyl and benzoyl, nitro, nitroso, as well as tetrahydropyranyl and methoxymethyl. Preferred examples of the ar(lower)alkyl include phenyl, benzyl, 4-hydroxybenzyl and 4-hydroxymethylbenzyl. This is also the case in the following description.

The aryl" for $R_3$ includes phenyl and condensed polycyclic hydrocarbons such as naphthyl and anthranyl. In this case, the aromatic ring may be substituted with at least one of the substituents consisting of a lower alkyl, hydroxy (lower)alkyl, hydroxyl and protected hydroxyl. In general, the aromatic ring is suitable for the present invention because it takes on a planar structure and thereby shows small steric hindrance and high hydrophobic property. Phenyl is a preferred aromatic ring. The lower alkyl per se or the lower alkyl in the hydroxy(lower)alkyl as a substituent on the aromatic ring is preferably a C1-3 alkyl having small steric hindrance such as methyl, ethyl or propyl.

In general formula (I), the total number of carbon atoms of the substituents represented by $R_4$, P and Q is preferably 2-20 for the purpose of improved molecular stability and hydrophobic property.

The "lower alkyl" for $R_4$, includes C1-10 straight- and branched-chain alkyls, and cyclic alkyls such as methyl, ethyl, propyl, isopropyl, cyclopropyl, butyl, isobutyl, tert-butyl, pentyl, isopentyl, cyclopentyl, hexyl, isohexyl, cyclohexyl, heptyl, 5-methylhexyl, cycloheptyl, octyl, 6-methylheptyl, nonyl, 7-methyloctyl, decyl and 8-methylnonyl. Considering steric hindrance, C1-6 alkyls are preferred. Straight-chain alkyls are preferred to branched chain and cyclic ones. Both P and Q are methyl. The "lower alkenyl" for $R_4$ includes C2-10 straight- and branched-chain alkenyls such as vinyl and propenyl. Considering steric hindrance, C2-6 alkenyls are preferred.

The "lower alkynyl" for $R_4$ includes C2-10 straight- and branched-chain alkynyls such as ethynyl, propynyl and butynyl. Considering steric hindrance, C2-6 alkynyls are preferred.

The lower alkyl moiety of the hydroxy(lower)alkyl, nitrooxy (lower)alkyl or nitrosooxy(lower)alkyl for $R_4$ includes C1-10 lower alkyls. Preferred are C1-6 alkyls. Examples of the hydroxy(lower)alkyl include hydroxymethyl, 1-hydroxyethyl, 2-hydroxyethyl and 3-hydroxypropyl. Preferred examples of the nitrooxy(lower)alkyl include 1-nitrooxyethyl. Preferred examples of the nitrosooxy(lower)alkyl include 1-nitrosooxyethyl.

The lower alkyl moiety of the "amino(lower)alkyl" for $R_4$ includes C1-10 lower alkyls. Preferred are C1-6 alkyls. Examples of the amino(lower)alkyl include aminomethyl, 1-aminoethyl, 2-aminoethyl, 3-aminopropyl and piperidine. The amino group in the amino(lower)alkyl may be modified with an alkyl. Preferred examples of the modified case include 1-N,N-dimethylaminomethyl, 2-N,N-dimethylaminoethyl, 3-N,N-dimethylaminopropyl, 1-N,N-diethylamino-methyl, 2-N,N-diethylaminoethyl and 3-N,N-diethylaminopropyl.

The "heterocycle-substituted lower alkyl" for $R_4$ includes lower alkyls substituted with a 5-6 membered heterocycle containing at least one hetero atom such as nitrogen. Preferred examples include piperidine(lower)alkyls such as piperidinemethyl and piperidineethyl, and piperazine(lower)alkyls such as piperazinemethyl and piperazineethyl. The heterocycle-substituted lower alkyl also includes lower alkyls substituted with an unsaturated condensed heterocycle containing at least one hetero atom such as nitrogen. Preferred examples include pyridine(lower)alkyls such as pyridinemethyl and pyridineethyl, and indole(lower)alkyls such as indolemethyl and indoleethyl.

The "ar(lower)alkyl" for $R_4$ includes phenylalkyls such as benzyl and phenethyl. In this case, the aryl moiety

such as phenyl may have lower alkyl, halogen, nitro, amino, carboxyl, hydroxy(lower)alkyl, hydroxyl or protected hydroxyl. The lower alkyl per se or the lower alkyl in the hydroxy(lower)alkyl is preferably a C1-3 alkyl having small steric hindrance such as methyl, ethyl or propyl. Specific examples include benzyl, phenethyl and phenylpropyl which may have methyl, ethyl, propyl, isopropyl, butyl, chloro, fluoro, methoxy, ethyoxy, hydroxy, hydroxymethyl, amino, carboxyl, nitro or dimethylamino independently as substituents at the o, p and/or m positions. Preferred are benzyl, phenethyl, phenylpropyl, 4-hydroxybenzyl, 3-hydroxybenzyl, 4-methoxybenzyl, 4-fluorobenzyl, 4-chlorobenzyl, 4-hydroxyphenethyl, 3-hydroxyphenethyl, 4-methoxyphenethyl, 4-fluorophenethyl and 4-chlorophenethyl.

The "ar(lower)alkenyl" for $R_4$ includes phenylalkenyls such as cinnamyl and styryl. In this case, the aryl moiety such as phenyl may have a lower alkyl, halogen, nitro, amino, carboxyl, hydroxy(lower)alkyl, hydroxyl or protected hydroxyl. The lower alkyl per se or the lower alkyl in the hydroxy(lower)alkyl is preferably a C1-3 alkyl having small steric hindrance such as methyl, ethyl or propyl. Specific examples include cinnamyl and styryl which may have methyl, ethyl, propyl, isopropyl, butyl, chloro, fluoro, methoxy, ethyoxy, hydroxy, hydroxymethyl, amino, carboxyl, nitro or dimethylamino independently as substituents at the o, p and/or m positions. Preferred are cinnamyl, styryl, 4-hydroxycinnamyl, 3-hydroxycinnamyl, 4-methoxycinnamyl, 4-fluorocinnamyl, 4-chlorocinnamyl, 4-hydroxystyryl, 3-hydroxystyryl, 4-methoxystyryl, 4-fluorostyryl and 4-chlorostyryl.

The "ar(lower)alkynyl" for $R_4$ includes phenylalkynyls such as phenylethynyl and phenylpropynyl. In this case, the aryl moiety such as phenyl may have a lower alkyl, halogen, nitro, amino, carboxyl, hydroxy(lower)alkyl, hydroxyl or protected hydroxyl. The lower alkyl per se or the lower alkyl in the hydroxy(lower)alkyl is preferably a C1-3 alkyl having small steric hindrance such as methyl, ethyl or propyl. Specific examples include phenylethynyl and phenylpropynyl which may have methyl, ethyl, propyl, isopropyl, butyl, chloro, fluoro, methoxy, ethyoxy, hydroxy, hydroxymethyl, amino, carboxyl, nitro or dimethylamino independently as substituents at the o, p and/or m positions. Preferred are phenylethynyl, phenylpropynyl, 4-hydroxyethynyl, 3-hydroxyethynyl, 4-methoxyethynyl, 4-fluoroethynyl, 4-chloroethynyl, 4-hydroxypropynyl, 3-hydroxypropynyl, 4-methoxypropynyl, 4-fluoropropynyl and 4-chloropropynyl.

The "aryl" for $R_4$ includes phenyl and condensed polycyclic hydrocarbons such as naphthyl and anthranyl. In this case, the aromatic ring may be substituted with a lower alkyl, halogen, nitro, amino, carboxyl, hydroxy(lower)alkyl, hydroxyl or protected hydroxyl. In general, the aromatic ring is suitable for the present invention because it takes on a planar structure and thereby shows small steric hindrance and high hydrophobic property. Phenyl is a preferred aromatic ring. The lower alkyl per se or the lower alkyl in the hydroxy(lower)alkyl as a substituent on the aromatic ring is preferably a C1-3 alkyl having small steric hindrance such as methyl, ethyl or propyl.

The "heterocycle" for $R_4$ includes any heterocyclic functional groups. Specific examples include pyridyl, furyl, pyrrolyl, thiophene, oxazolyl, thiazolyl, imidazolyl, pyrazolyl, tetrazolyl, triazolyl, indolyl, benzothiazolyl, benzimidazolyl, benzoxazolyl, coumaryl, carbazolyl, pyranyl, pyronyl, quinolyl, isoquinolyl, pyrimidyl, pyrazinyl, piperidyl, piperazyl and tetrahydrofuryl. In this case, the heterocycle may be substituted with a lower alkyl, halogen, nitro, amino, carboxyl, hydroxy(lower)alkyl, hydroxyl or protected hydroxyl. The lower alkyl per se or the lower alkyl in the hydroxy(lower)alkyl is preferably a C1-3 alkyl having small steric hindrance such as methyl, ethyl or propyl. Preferred examples of the heterocycle include pyridyl, piperidyl and furyl.

The lower alkyl moiety of the "lower alkyl substituted with a cycloalkyl having a 3-8 membered ring" for $R_4$ includes C1-10 alkyls. Preferred are C1-3 alkyls. The lower alkyl substituted with a suitable cycloalkyl includes cyclohexylmethyl, cyclopentylmethyl, cyclohexylethyl, cyclopentylethyl, cyclohexylpropyl and cyclopentylpropyl. The ring moiety of the lower alkyl substituted with a cycloalkyl having a 3-8 membered ring may have a lower alkyl, halogen, nitro, amino, carboxyl, hydroxy(lower)alkyl, hydroxyl or protected hydroxyl. The lower alkyl per se or the lower alkyl in the hydroxy(lower)alkyl is preferably a C1-3 alkyl having small steric hindrance such as methyl, ethyl or propyl. More specifically, preferred are 4-hydroxycyclohexylmethyl, 3-hydroxy-cyclohexylmethyl, 4-methoxycyclohexylmethyl, 4-fluorocyclohexylmethyl, 4-chlorocyclohexylmethyl, 3-hydroxycyclopentylmethyl, 3-methoxycyclopentyl-methyl, 3-fluorocyclopentylmethyl, 3-chlorocyclo-pentylmethyl, 4-hydroxycyclohexylethyl, 3-hydroxy-cyclohexylethyl, 4-methoxycyclohexylethyl, 4-fluoro-cyclohexylethyl, 4-chlorocyclohexylethyl, 3-hydroxy-cyclopentylethyl, 3-methoxycyclopentylethyl, 3-fluorocyclopentylethyl, 3-chlorocyclopentylethyl, 4-hydroxycyclohexylpropyl, 3-hydroxycyclohexylpropyl, 4-methoxycyclohexylpropyl, 4-fluorocyclohexylpropyl, 4-chlorocyclohexylpropyl, 3-hydroxycyclopentylpropyl, 3-methoxycyclopentylpropyl, 3-fluorocyclopentyl-propyl and 3-chlorocyclopentylpropyl.

The lower alkynyl moiety of the "lower alkynyl substituted with a cycloalkyl having a 3-8 membered ring" for $R_4$ includes C1-10 alkynyls. Preferred are C2-3 alkynyls. The cycloalkyl(lower)alkynyl includes cyclohexylethynyl and cyclohexylpropynyl.

The ring moiety of the lower alkynyl substituted with a cycloalkyl having a 3-8 membered ring may have a lower alkyl, halogen, nitro, amino, carboxyl, hydroxy(lower)alkyl, hydroxyl or protected hydroxyl. The lower alkyl per se or the lower alkyl in the hydroxy(lower)alkyl is preferably a C1-3 alkyl having small steric hindrance such as methyl, ethyl or propyl. More specifically, preferred are 4-hydroxycyclohexylethynyl, 3-hydroxycyclohexyl-ethynyl, 4-methoxycyclohexylethynyl, 4-fluorocyclo-hexylethynyl, 4-chlorocyclohexylethynyl, 3-hydroxy-cyclopentylethynyl, 3-methoxycyclopentylethyl, 3-fluorocyclopentylethynyl, 3-chlorocyclopentylethynyl, 4-hydroxycyclohexylpropynyl, 3-hydroxycyclohexyl-propynyl, 4-methoxycyclohexylpropynyl, 4-fluoro-cyclohexylpropynyl, 4-chlorocyclohexylpropynyl, 3-hydroxycyclopentylpropynyl, 3-methoxycyclopentylpropynyl, 3-fluorocyclopentylpropynyl and 3-chlorocyclopentylpropynyl.

The lower alkenyl moiety of the "lower alkenyl substituted with a cycloalkyl having a 3-8 membered ring" for $R_4$ includes C1-10 alkenyls. Preferred are C2-3 alkenyls. The cycloalkyl(lower)alkenyl includes 2-cyclohexylvinyl, 2-cyclopentylvinyl, 3-cyclohexyl-2-propenyl and 3-cyclopentyl-2-propenyl. The ring moiety of the lower alkenyl substituted with a cycloalkyl having a 3-8 membered ring may have a lower alkyl, halogen, nitro, amino, carboxyl, hydroxy(lower) alkyl, hydroxyl or protected hydroxyl. The lower alkyl per se or the lower alkyl in the hydroxy(lower)alkyl is preferably a C1-3 alkyl having small steric hindrance such as methyl, ethyl or propyl. More specifically, preferred are 2-(4-hydroxy)-cyclohexylvinyl, 2-(3-hydroxy)-cyclohexylvinyl, 2-(4-methoxy)-cyclohexylvinyl, 2-(4-fluoro)-cyclohexylvinyl, 2-(4-chloro)-cyclohexylvinyl, 2-(3-hydroxy)-cyclopentylvinyl, 2-(3-methoxy)-cyclopentylvinyl, 2-(3-fluoro)-cyclopentylvinyl, 2-(3-chloro)-cyclopentylvinyl, 3-(4-hydroxy)-cyclohexyl-2-propenyl, 3-(3-hydroxy)-cyclohexyl-2-propenyl, 3-(4-methoxy)-cyclohexyl-2-propenyl, 3-(4-fluoro)-cyclohexyl-2-propenyl, 3-(4-chloro)-cyclohexyl-2-propenyl, 3-(3-hydroxy)-cyclopentyl-2-propenyl, 3-(3-methoxy)-cyclopentyl-2-propenyl, 3-(3-fluoro)-cyclopentyl-2-propenyl and 3-(3-chloro)-cyclopentyl-2-propenyl.

The lower alkyl moiety of the "lower alkyloxy" for $R_4$ includes C1-10 lower alkyls. Preferred are C1-6 alkyls. The lower alkyloxy includes methoxy, ethoxy, propoxy, isopropoxy, butoxy and isobutoxy.

The "physiologically cleavable carboxyl-protecting group" for $R_5$ includes any carboxyl-protecting groups which are known to be physiologically cleavable. Specific examples include protective groups which protect the carboxyl group in binding modes as described in Development of Medicines", vol. 13, Drug Delivery", p. 116, Table 2.29, Jin Sezaki ed., Hirokawa Shoten, 1989, July. Examples of the protective groups are alkoxy groups capable of forming esters such as methyl esters, ethyl esters, propyl esters, isopropyl esters and butyl esters, amino acid residues having a free amino group, protected amino acid residues thereof and 1-acyloxyalkoxides. In particular, ethoxy, propoxy, isopropoxy, butoxy and acyloxymethoxy are preferred.

X is nitrogen or CH and preferably CH.

$Y_1$ and $Y_2$ are each independently the atom or group, as defined above, preferably H or halogen. The "lower alkyl" for $Y_1$ and $Y_2$ is preferably a C1-3 alkyl having small steric hindrance, i.e., methyl, ethyl or propyl. The halogen" for $Y_1$ and Y2 is preferably fluorine or chlorine. The "lower alkoxy" for $Y_1$ and $Y_2$ includes methoxy and ethoxy. Preferred is methoxy having small steric hindrance. The "lower acyloxy" for $Y_1$ and $Y_2$ includes acetoxy, propionyloxy and benzoyloxy. Preferred is acetoxy having small steric hindrance. The "acyl" for $Y_1$ and Y2 includes formyl, acetyl, propionyl and benzoyl. Preferred is acetyl. The "lower alkoxycarbonyl" for $Y_1$ and $Y_2$ includes methoxycarbonyl, ethoxycarbonyl, propoxycarbonyl and butoxycarbonyl. Preferred are methoxycarbonyl and ethoxycarbonyl. Preferred examples are 2-methyl, 3-methyl, 2-chloro, 2,6-dichloro, 2-fluoro, 2,6-difluoro, 2-hydroxy, 2-methoxy, 2-acetoxy, 2-acetyl, 2-benzoyl, 2-carboxyl, 2-methoxycarbonyl, 2-nitro, 3-nitro and 2-trifluoromethyl.

m is an integer of 0 to 2 and most preferably 1.

Among the compounds of general formula (V), compounds of the following general formula (II) wherein P and Q are both methyl, m is 1 and X is CH are preferred:

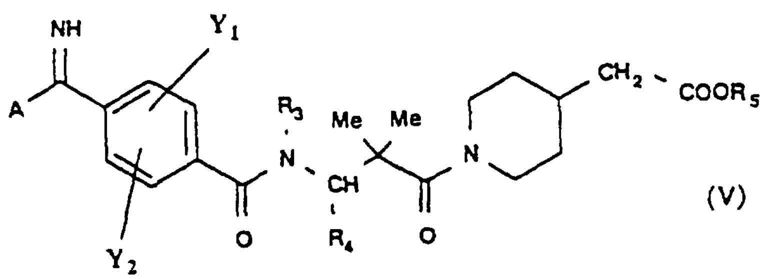

(V)

wherein A, $Y_1$, $Y_2$, $R_3$, $R_4$ and $R_5$ are as defined above.

Preferred examples of the compounds of general formula (I) are ethyl N-(N-(4-(4-morpholinoimidoyl)benzoyl-β-ethyl-α,α-dimethyl-β-alanyl)-4-piperidineacetate, N-(N-(4-(4-morpholinoimidoyl)benzoyl-β-ethyl-α,α-dimethyl-β-alanyl)-4-piperidineacetic acid, ethyl N-(N-(4-(4-morpholinoimidoyl)-2-fluorobenzoyl)-β-ethyl-α,α-dimethyl-β-alanyl)-4-piperidineacetate, N-(N-(4-(4-morpholinoimidoyl)-2-fluorobenzoyl)-β-ethyl-α,α-dimethyl-β-alanyl)-4-piperidineacetic acid, ethyl N-(N-(4-(N,N-diethylaminoimidoyl)-2-fluorobenzoyl)-β-ethyl-α,α-dimethyl-β-alanyl)-4-piperidineacetate, N-(N-(4-(N,N-diethylaminoimidoyl)-2-fluorobenzoyl)-β-ethyl-α,α-dimethyl-β-alanyl)-4-piperidineacetic acid, ethyl N-(N-(4-(1-piperidinoimidoyl)-2-fluorobenzoyl)-β-ethyl-α,α-dimethyl-β-alanyl)-4-piperidineacetate, N-(N-(4-(1-piperidinoimidoyl)-2-fluorobenzoyl)-β-ethyl-α,α-dimethyl-β-alanyl)-4-piperidineacetic acid, ethyl N-(N-(4-(1-pyrrilidinoimidoyl)-2-fluorobenzoyl)-β-ethyl-α,α-dimethyl-β-alanyl)-4-piperidineacetate, N-(N-(4-(1-pyrrolidinoimidoyl)-2-fluorobenzoyl)-β-ethyl-α,α-dimethyl-β-alanyl)-4-piperidineacetic acid, ethyl N-(N-(4-(1-4-piperidonoimidoyl)-2-fluorobenzoyl)-β-ethyl-α,α-dimethyl-β-alanyl)-4-piperidineacetate, N-(N-(4-(1-4-piperidonoimidoyl)-2-fluorobenzoyl)-β-ethyl-α,α-dimethyl-β-alanyl)-4-piperidineacetic acid, ethyl N-(N-(4-(4-hydroxy-1-piperidinoimidoyl)-2-fluorobenzoyl)-β-ethyl-α,α-dimethyl-β-alanyl)-4-piperidineacetate, N-(N-(4-(4-hydroxy-l-piperidinoimidoyl)-2-fluorobenzoyl)-β-ethyl-α,α-dimethyl-β-alanyl)-4-piperidineacetic acid, ethyl N-(N-(4-(3-thiazolidinoimidoyl)-2-fluorobenzoyl)-β-ethyl-α,α-dimethyl-β-alanyl)-4-piperidineacetate, N-(N-(4-(3-thiazolidinoimidoyl)-2-fluorobenzoyl)-β-ethyl-α,α-dimethyl-β-alanyl)-4-piperidineacetic acid, ethyl N-(N-(4-(1-hexamethyleneiminoimidoyl)-2-fluorobenzoyl)-β-ethyl-α,α-dimethyl-β-alanyl)-4-piperidineacetate, N-(N-(4-(1-hexamethyleneiminoimidoyl)-2-fluorobenzoyl)-β-ethyl-α,α-dimethyl-β-alanyl)-4-piperidineacetic acid, ethyl N-(N-(4-(N-thiomorpholinoimidoyl)-2-fluorobenzoyl)-β-ethyl-α,α-dimethyl-β-alanyl)-4-piperidineacetate, N-(N-(4-(N-thiomorpholinoimidoyl)-2-fluorobenzoy)-β-ethyl-α,α-dimethyl-β-alanyl)-4-piperidineacetic acid, ethyl N-(N-(4-(4-methyl-1-piperazinoimidoyl)-2-fluorobenzoyl)-β-ethyl-α,α-dimethyl-β-alanyl)-4-piperidineacetate, N-(N-(4-(4-methyl-1-piperazinoimidoyl)-2-fluorobenzoyl)-β-ethyl-α,α-dimethyl-β-alanyl)-4-piperidineacetic acid, ethyl N-(N-(4-(4-phenyl-1-piperazino)imidoyl)-2-fluorobenzoyl)-β-ethyl-α,α-dimethyl-β-alanyl)-4-piperidineacetate, N-(N-(4-(4-phenyl-1-piperazinoimidoyl)-2-fluorobenzoyl)-β-ethyl-α,α-dimethyl-β-alanyl)-4-piperidineacetic acid, ethyl N-(N-(4-(4-(4-fluorophenyl)-1-piperazinoimidoyl)-2-fluorobenzoyl)-β-ethyl-α,α-dimethyl-β-alanyl)-4-piperidineacetate, N-(N-(4-(4-(4-fluorophenyl)-1-piperazinoimidoyl)-2-fluorobenzoyl)-β-ethyl-α,α-dimethyl-β-alanyl)-4-piperidineacetic acid, benzyl N-(N-(4-(4-morpholinoimidoyl)benzoyl-β-phenyl-α,α-dimethyl-β-alanyl)-4-piperidineacetate, N-(N-(4-(4-morpholinoimidoyl)benzoyl-β-phenyl-α,α-dimethyl-β-alanyl)-4-piperidineacetic acid, methyl N-(N-(4-(1-pyrrolidinoimidoyl)-2-fluorobenzoyl)-β-ethyl-α,α-dimethyl-β-alanyl)-4-piperidineacetate, N-(N-(4-(N-tetrahydroisoquinolinoimidoyl)-2-fluorobenzoyl)-β-ethyl-α,α-dimethyl-β-alanyl)-4-piperidineacetic acid, N-(N-(4-(4-phenyl-1-piperadinoimidoyl)-2-fluorobenzoyl)-β-(m-hydroxyphenyl)-α,α-dimethyl-β-alanyl)-4-piperidineacetic acid, N-(N-(4-(4-fluoro-1-piperidineimidoyl)-2-fluorobenzoyl)-β-(3,5-difluorophenyl)-α,α-dimethyl-β-alanyl)-4-piperidinacetic acid, N-(N-(4-(diphenylaminoimidoyl)-2-fluorobenzoyl)-β-methyl-α,α-dimethyl-β-alanyl)-4-piperidineacetic acid, N-(N-(4-(N-indolylimidoyl)-2-fluorobenzoyl)-β-n-butyl-α,α-dimethyl-β-alanyl)-4-piperidineacetic acid, N-(N-(4-phenyl-1-piperazinoimidoyl)-2-fluorobenzoyl)-β-ethyl-α,α-dimethyl-β-alanyl)-4-piperadineacetic acid, N-(N-(4-(4-(2-pyrimidyl)-1-piperazinoimidoyl)-2-fluorobenzoyl)-β-cyclopropyl-α,α-dimethyl-β-alanyl)-4-piperidineacetic acid, N-(N-(4-(4-methyl-1-homopiperazinoimidoyl)-2-chlorobenzoyl)-β-n-butyl-α,α-dimethyl-β-alanyl)-4-piperidineacetic acid, N-(N-(4-(N-(4,4'-dipiperidino)imidoyl)-benzoyl)-β-ethyl-α,α-dimethyl-β-alanyl)-4-piperidineacetic acid, N-(N-(4-(4-(3-trifluorophenyl)-1-piperazinoimidoyl)-2-fluorobenzoyl)-β-ethyl-α,α-dimethyl-β-alanyl)-4-piperidineacetic acid, N-(N-(4-(4-(2-quinolyl)-1-piperazinoimidoyl)-2-methoxybenzoyl)-β-phenyl-α,α-dimethyl-β-alanyl)-4-pyperidineacetic acid, N-(N-(4-(N-(3-hydroxypyrrolidinoimidoyl)-2-fluorobenzoyl)-β-ethyl-α,α-dimethyl-β-alanyl)-4-piperidineacetic acid, N-(N-(4-(N-cis-3,4-dihydroxypyrrolidinoimidoyl)-2-fluorobenzoyl)-β-phenyl-α,α-dimethyl-β-alanyl)-4-piperidineacetic acid, N-(N-(4-(N-3-hydroxypiperidinoimidoyl)-2-fluorobenzoyl)-β-ethyl-α,α-dimethyl-β-alanyl)-4-piperidineacetic acid, N-(N-(4-(N-cis-3,4-dihydroxyplperidinoimidoyl)-2-fluorobenzoyl)-β-ethyl-α,α-dimethyl-β-alanyl)-4-piperidineacetic acid, N-(N-(4-(N-(4-(methylamino)piperidinoimidoyl)-2-fluorobenzoyl)-β-ethyl-α,α-dimethyl-β-alanyl)-4-piperidineacetic acid, N-(N-(4-(N-methylpipeconylimidoyl)-2-fluorobenzoyl)-β-ethyl-α,α-dimethyl-β-alanyl)-4-piperidineacetic acid, N-(N-(4-(N-methylnipecotinylimidoyl)-2-fluorobenzoyl)-β-methyl-α,α-dimethyl-β-alanyl)-4-piperidineacetic acid, N-(N-(4-(N-(2,2,6,6-pentamethylpiperidino)imidoyl)benzoyl)-β-ethyl-α,α-dimethyl-β-alanyl)-4-piperidineacetic acid, N-(N-(4-(1-(1,4,8,11-tetraazacyclotetradecanyl)imidoyl)-2-fluorobenzoyl)-β-(4-hydroxyphenyl)-α,α-dimethyl-β-alanyl)-4-piperidineacetic acid, N-(N-(4-(N-tetrahydroisoquinolinylimidoyl)-2-fluorobenzoyl)-β-2-morpholinyl-α,α-dimethyl-β-alanyl)-4-piperidineacetic acid, N-(N-(4-(N-oxazolidinoylimidoyl)-2-fluorobenzoyl)-β-3-hydroxyphenyl-α,α-dimethyl-β-alanyl)-4-piperidineacetic acid, N-(N-(4-(n-4,5-dihydrooxazolidinoylimidoyl)-2-fluorobenzoyl)-β-ethyl-α,α-dimethyl-β-alanyl)-4-piperidineacetic acid, N-(N-(4-(N-thiazolydinoylimidoyl)-2-fluorobenzoyl)-β-ethyl-α,α-dimethyl-β-alanyl)-4-piperidineacetic acid, N-(N-(4-(N-4,5-dihydro-thiazolidinoylimidoyl)-2-fluorobenzoyl)-β-methyl-α,α-dimethyl-β-alanyl)-4-piperidineacetic acid, N-(N-(4-(N-3-methoxy-piperidinylimidoyl)-2-fluorobenzoyl)-β-ethyl-α,α-dimethyl-β-alanyl)-4-piperidineacetic acid, N-(N-(4-(1,2-dihydro-triazinylimidoyl)-2-fluorobenzoyl)-β-ethyl-α,α-dimethyl-β-alanyl)-4-piperidineacetic acid, N-(N-(4-18-crown-6-morpholinoimidoyl)-2-fluorobenzoyl)-β-ethyl-α,α-dimethyl-β-alanyl)-4-piperidineacetic acid, N-(N-(4-(azetidinylimidoyl)-2-fluorobenzoyl)-β-ethyl-α,α-dimethyl-β-alanyl)-4-piperidineacetic acid, N-(N-(4-(azetidinylimidoyl)-2-fluorobenzoyl)-β-ethyl-α,α-dimethyl-β-alanyl)-4-piperadineacetic acid, N-(N-(4-(2-hydroxy-piperidinylimidoyl)benzoyl)-β-ethyl-α,α-dimethyl-β-alanyl)-4-piperidineacetic acid, N-(N-(4-(N-imidazolylimidoyl)-2-fluorobenzoyl)-β-ethyl-α,α-dimethyl-β-alanyl)-4-piperidineacetic acid, N-(N-(4-(N-imidaozolylimidoyl)-2-fluorobenzoyl)-β-ethyl-α,α-dimethyl-β-alanyl)-4-piperidineacetic acid, N-(N-(4-(1-(4-hydroxypiperadinyl)imidoyl)-2-fluorobenzoyl)-β-ethyl-α,α-dimethyl-β-alanyl)-4-piperidineacetic acid, N-(N-(4-(l-cis-3,4-dihydroxypyrrolidinylimidoyl)-2-fluorobenzoyl)-β-ethyl-α,α-dimethyl-β-alanyl)-4-piperidineacetic acid, N-(N-(4-(N-pyrrolidinylimidoyl)-2-fluorobenzoyl)-β-ethyl-α,α-dimethyl-β-alanyl)-4-piperidineacetic acid and N-(N-(4-(N-3,4-dihydropyrrolidinylimidoyl)-2-fluorobenzoyl)-β-ethyl-α,α-dimethyl-β-alanyl)-4-piperidineacetic acid.

The pharmaceutically acceptable salts of the compounds of the present invention are conventional nontoxic salts. Exemplary salts are salts with bases and acid addition salts including salts with inorganic bases such as alkaline

metal salts (e.g., sodium salts and potassium salts), alkaline earth metal salts (e.g., calcium salts and magnesium salts) and ammonium salts; salts with organic bases such as organic amine salts (e.g, triethylamine salts, pyridine salts, picoline salts, ethanolamine salts, triethanolamine salts, dicyclohexylamine salts and N,N'-dibenzylethylenediamine salts); inorganic acid addition salts (e.g., hydrochlorides, hydrobromides, sulfates and phosphates); organic carboxylic acid or sulfonic acid addition salts (e.g., formates, acetates, propionates, trifluoroacetates, maleates, malates, tartrates, succinates, citrates, methanesulfonates, benzenesulfonates, p-toluenesulfonates and glycolates); and salts with basic or acidic amino acids (e.g., salts with arginine, aspartic acid and glutamic acid).

The compounds of the present invention can be prepared by synthesis. A method for preparing the compounds of the present invention will now be described in detail.

Three structural parts of the compounds are synthesized separately and combined to prepare the compounds. The three parts are (a) a substituted-4-amidinobenzoic part which is located on the left side of general formula (I), (b) a substituted β-amino acid residue part which is located in the middle, and (c) a piperidine or piperazine part having a carboxyl or carboxyalkyl group at the 4-position which is located on the right side.

If these units are commercially available, they are used with or without protecting functional groups which do not take part in the reaction to prepare the compounds of the present invention. If these units are not commercially available, they are synthesized by an appropriate method and then used to prepare the compounds of the present invention by a conventional method which is used in peptide chemistry as described below.

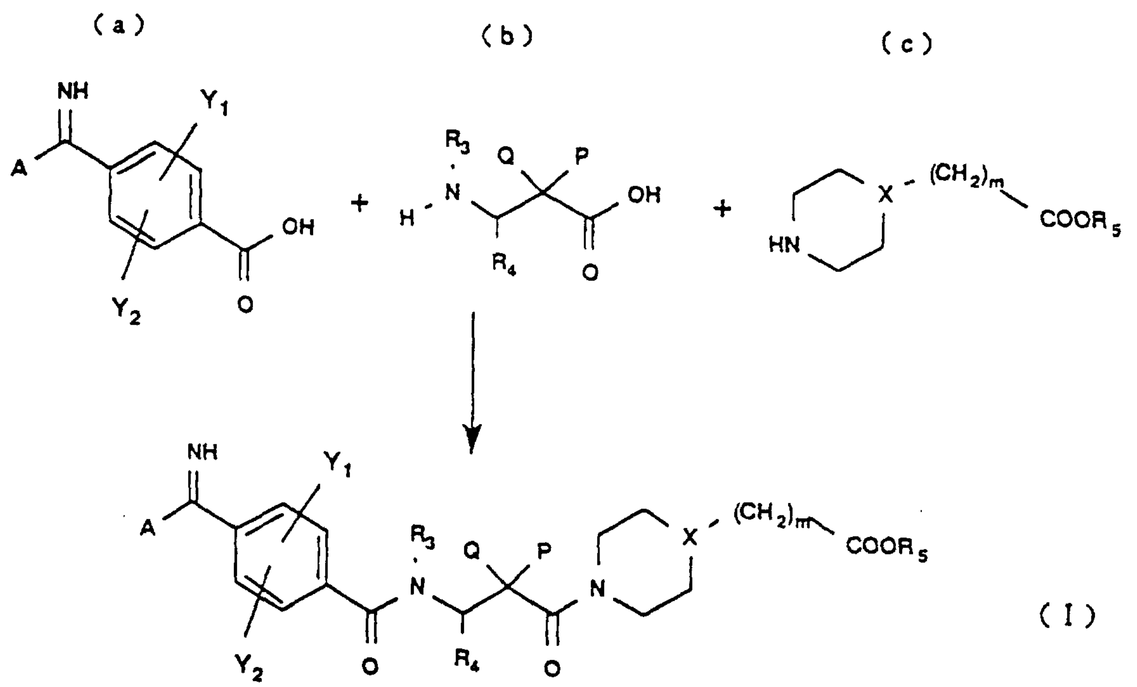

The compounds of the present invention can be also obtained by condensing synthetic precursors of the respective units and then derivatizing the obtained compounds to ones having desired functional groups.

Since the compounds of the present invention have two peptide bonds in the molecule, each amino acid-like unit can be synthesized either in a liquid or solid phase by any conventional methods used in peptide chemistry such as those described in Schroder and Luhke, "The Peptides" vol.1, Academic Press, New York, U.S.A. (1966), Nobuo Izumiya et al., The Fundamentals and Experiments of Peptide Synthesis", Maruzen (1985), and other references. These preparation methods may be a column or batch method.

The condensation methods for forming peptide bonds include, for example, the azide method, acid halide method, acid anhydride method, carbodiimide method, carbodiimide-additive method, active ester method, carbonyl imidazole method, redox method, enzymatic method and the method using Woodward's reagent K, HATU reagent or Bop reagent. In the case of performing a condensation reaction by a solid phase method, the acid anhydride method, carbodiimide method and active ester method may predominantly be used.

When a peptide chain is to be extended by the solid phase method, the C-terminal amino acid is coupled to a support such as a resin that is insoluble in organic solvents to be used. In this case, the resin may be modified

depending on the intended purpose by introducing a functional group for the purpose of bonding amino acids to the resin, by inserting a spacer between the resin and a functional group or by introducing a chain called handle" which can be cleaved in various positions under the conditions employed. Exemplary resins include halomethyl resins (such as chloromethyl resin), oxymethyl resin, 4-(oxymethyl)-phenylacetamide methyl resin, 4-(oxymethyl)-phenoxymethyl resin, resin for C-terminal amidation, and the like.

Prior to the condensation reaction, carboxyl, amino, hydroxyl and amidino groups that do not take part in the condensation reaction may be protected by conventional and known techniques. In contrast with this, carboxyl and amino groups that directly take part in the condensation reaction may be activated.

As protective groups for use in the protection of functional groups that do not take part in the condensation reaction of each unit, those which are commonly used in the field of organic chemistry, as described in Greene, Protective Groups in Organic Synthesis", John Wiley & Sons, Inc. (1981), can be used.

Exemplary protective groups for carboxyl group include commonly used and known protective groups such as various kinds of methyl ester, ethyl ester, benzyl ester, p-nitrobenzyl ester, t-butyl ester, cyclohexyl ester, and the like.

Exemplary protective groups for amino group include benzyloxycarbonyl, t-butoxycarbonyl, isobornyloxycarbonyl and 9-fluorenylmethoxycarbonyl groups.

Exemplary protective groups for hydroxyl group in the substituted β-amino acid residue containing a hydroxyl group include t-butyl, benzyl, trimethylsilyl and tetrahydropyranyl groups.

Exemplary protective groups for amidino group include benzyloxycarbonyl group.

Exemplary compounds with an activated carboxyl group include an acid anhydride corresponding to the carboxyl group; azide; active esters with pentafluorophenol, 2,4-dinitrophenol, cyanomethyl alcohol, p-nitrophenol, N-hydroxysuccinimide, N-hydroxy-5-norbornene-2,3-dicarboximide, N-hydroxyphthalimide and 1-hydroxybenzotriazole.

Exemplary compounds with an activated amino group include an amide phosphate corresponding to the amino group.

The condensation reaction for peptide synthesis is usually carried out in a solvent. Exemplary solvents include chloroform, dichloromethane, ethyl acetate, N,N-dimethylformamide, dimethyl sulfoxide, pyridine, dioxane, tetrahydrofuran, N-methylpyrrolidone, water, methanol and the like, and mixtures thereof. The condensation reaction can be carried out at a temperature of from -30 to 50°C as in the usual case.

The type of the deprotection reaction to be carried out in the peptide preparation process can be selected depending on the kind of protective groups provided that they can be eliminated without affecting the peptide bonds. Exemplary deprotection reactions include a treatment with an acid such as hydrogen chloride, hydrogen bromide, anhydrous hydrogen fluoride, methanesulfonic acid, trifluoromethane-sulfonic acid, trifluoroacetic acid, or a mixture thereof; a treatment with an alkali such as sodium hydroxide, potassium hydroxide, hydrazine, diethylamine, piperidine or the like; a treatment with sodium in liquid ammonia; reduction with palladium on carbon; a silylation treatment with trimethylsilyl triflate, trimethylsilyl bromide or the like. In the above deblocking reaction with an acid or silylation agent, cation-trapping agents such as anisole, phenol, cresol, thioanisole and ethanedithiol are preferably added to carry out the deblocking reaction effectively.

The compounds synthesized by the solid phase method can be cleaved from the solid phase by conventional methods. Exemplary methods for cleaving the compounds include treatments with the acid or silylation agents described above.

The compounds of the present invention thus prepared can be separated and purified in a conventional and known manner after the end of the series of reactions described above. For example, extraction, partition, reprecipitation, recrystallization, column chromatography and the like can be used to obtain the compounds in a more purified form.

The method of synthesizing each unit will now be described. A substituted-4-amidinobenzoic acid of Unit (a) can be used as such in the condensation of the unit. Alternatively, it can be converted to 4-cyanobenzoic acid and then condensed, followed by conversion of the cyano group to a substituted amidino group (see the following formula).

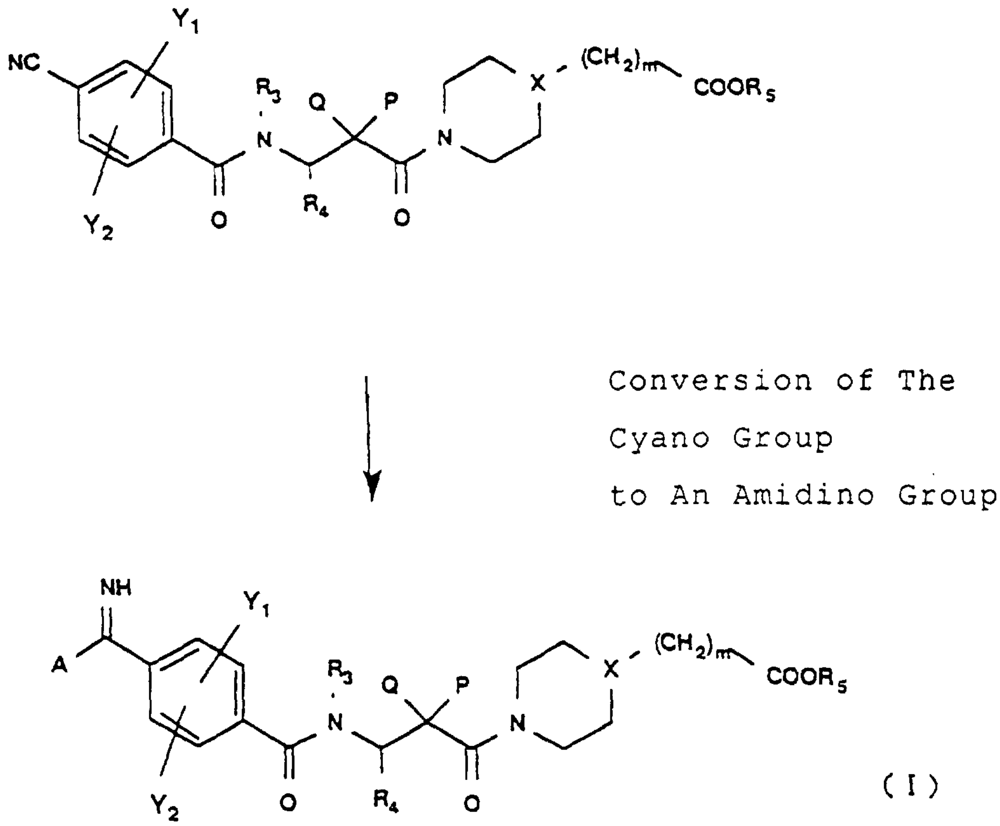


(I)

Unit (b) can be obtained by general methods for synthesizing substituted β-amino acids, for example, the method described in "Enantioselective Synthesis of β-amino acids", Eusebio Juaristi, et al., Aldrichimica Acta, Vol.27, No.1, pp.3-11, 1994 and the references cited therein. Alternatively, since β-lactam derivatives provide substituted β-amino acids upon cleavage of the lactam rings by hydrolysis, Unit (b) can be obtained easily at low cost by the preparation of β-lactam and subsequent hydrolysis as described in "A Synthetic Chemistry Review of Recent Advances in the Reaction of β-lactam Ring Formation", Koichi Imai, Vol.50 of "Organic Synthetic Chemistry", No.2, pp.112-130, 1992, 'The Ester Enolate-Imine Condensation Route to beta-Lactams", David J. Hart and Deok-Chan Ha, Chemical Reviews, Vol.89, No.7, pp.1447-1465, 1989 and the references cited therein. Unit (c) can be obtained easily by reducing the corresponding 4-carboxyalkylpyridine or by oxidizing the hydroxyl group of a 4-hydroxyalkylpyridine having the same number of side chain carbon atoms including the carbon atoms of the carboxyl group and reducing the pyridine ring.

The compounds of the present invention can be used effectively as platelet aggregation-inhibiting agents in treating and preventing various diseases caused primarily or secondarily by platelet aggregation. In particular, they are useful as agents for inhibiting or preventing the arterial occlusion caused by thrombus formation as in cardiac infarction and cerebral infarction. Furthermore, they are useful as agents for inhibiting: acute reocclusion after percutaneous transluminal coronary angioplasty (PTCA) is applied to stenosed coronary arteries in patients with angina or myocardial infarction; reocclusion caused by reactivated platelets released from thrombus at the time of applying thrombolytic therapy using a fibrinolytic agent such as urokinase to the arterial thrombus; and blood coagulation during medical treatments involving the extracorporeal circulation of blood.

The compounds of the present invention can also be used as cell adhesion-inhibiting agents, antiinflammatory agents, anti-rheumatic agents, anti-osteoporosis agents and cancer metastasis-inhibiting agents.

When the compounds of the present invention thus prepared are used as active ingredients of platelet aggregation-inhibiting agents, they or their salts are formulated together with a solid or liquid pharmaceutically acceptable carrier or diluent, that is, an excipient, stabilizer, etc. In the pharmaceutical preparation, the ratio of the active ingredient to the carrier can be varied in the range of 1 to 90% by weight.

The preparation may be in the form of granules, fine granules, powders, tablets, capsules, pills, liquids and solutions. The preparations may be orally administered in the form of bulk powders or they can be administered intravenously, intramuscularly or subcutaneously as injections. The injections may be prepared from powders of the compounds of the present invention or salts thereof just before use.

An organic or inorganic, solid or liquid pharmaceutically acceptable carrier or diluent suitable for oral, enteral or parenteral administration can be used to prepare the platelet aggregation-inhibiting agents of the present invention. Water, gelatin, lactose, starch, magnesium stearate, talc, animal fats and oils, vegetable fats and oils, benzyl alcohol, gums, polyalkylene glycol, petroleum resins, coconut oil, lanolin, and all other carriers for medicines can be used as carriers or diluents for the platelet aggregation-inhibiting agents of the present invention. Stabilizers, wetting agents, emulsifying agents, and salts for adjusting the osmolarity or pH of the preparation can appropriately be used as adjuvants.

If necessary, the platelet aggregation-inhibiting agents of the present invention may contain other pharmaceutically active ingredients such as other kinds of platelet aggregation-inhibiting agents.

In the case where the platelet aggregation-inhibiting agents are used in the form of granules, fine granules, powders, tablets or capsules, the content of the active ingredient is preferably in the range from 5 to 80% by weight. In the case where the platelet aggregation-inhibiting agents are used in the form of liquids and solutions, the content of the active ingredient is preferably in the range from 1 to 30% by weight. Furthermore, in the case where the platelet aggregation-inhibiting agents are used in the form of injections, the content of the active ingredient is preferably in the range from 1 to 10% by weight.

When the platelet aggregation-inhibiting agents are to be administered orally, the clinical dose of the active ingredient is preferably in the range from 100 to 1000 mg per day for each adult patient, which can be varied depending on the age of the patient, severity of the diseases to be treated and the like. The platelet aggregation-inhibiting agents can be administered in the aforementioned daily dose either once a day, or twice or three times a day at suitable intervals. In the case of injections, the dose of the active ingredient is preferably in the range from one to several hundreds milligrams per injection for each adult patient. The administration can be conducted stepwise by means of injection or continued over time by means of drip infusion and the like. When the compounds or salts of the present invention are used for extracorporeal circulation, they can be used in the form of injections. The dose thereof is the same as in the case of the platelet aggregation-inhibiting agents.

As shown in the Comparative Examples described below, β-amino acid derivatives which are mono-substituted at the α positions do not generally have high biological activity. If substituents are introduced at the β positions of these derivatives, their biological activity greatly decreases. In contrast, the β-amino acid derivatives of the present invention which are substituted at the α positions with two lower alkyls are generally about 10 times more biologically active than unsubstituted β-amino acid derivatives. Moreover, the introduction of substituents at the β positions causes a great increase in the biological activity. This would be because the β-amino acid derivative of the present invention has the spatial position of a substituent at the β position fixed at the site where it can interact with a receptor by inserting the substituent at the β position between two substituents at the α position and the amide group.

The β-amino acid residues of the compounds of the present invention are formed by converting the moiety of peptide-bond forming amino acid residues from α-amino acid residues, which generally constitute proteins in the body, to nearly non-native β-amino acid residues. Such a conversion can greatly increase the in vivo stability of peptide bonds against proteolytic enzymes, thereby inhibits the degradation of the compounds of the present invention in the body and prolongs the working time of drugs. The introduction of substituents in the β-amino acid residues further increases the stability against proteolytic enzymes and enhances the hydrophobic nature of the molecules, whereby the poor bioavailability of peptide-bond-containing compounds like those of the present invention which results from the hydrophilic nature of peptide bonds is modified such as to increase the bioavailability.

In order to antagonize the action of fibrinogen receptors, compounds should have both a basic and an acidic site, keeping at a certain spatial distance in the molecule, and these sites should bind to the fibrinogen receptors. In the compounds of the present invention, the basic site is a substituted amidino group and the acidic site is a fatty acid residue at the 4 position of the piperidine ring. Basically, any substituents that are not bulky enough to inhibit the binding of the two aforementioned receptor-recognizing sites to the receptor and which improve the bioavailability due to increased in vivo stability and hydrophobicity can be employed as substituents of the β-amino acid residues. In order to have high platelet aggregation-inhibiting activity, the substituents described herein are preferred for the following reasons. The substituents described herein have a hydrophobic nature and thereby provide new sites of interaction with the receptor. As a result, the receptor-binding force of the compounds of the present invention is further increased. In addition, the introduction of the substituents described herein regulates the motility of the compounds having straight-chain structures with a great degree of freedom and fixes the steric molecular structures so that the steric structures required for the development of high biological activity can be held stable and the receptor-binding force is increased. Hence, the introduction of those substituents is very important for improving the usefulness of the compounds.

The bioavailability of a compound is influenced by the nature of the functional groups in its molecule. An amidino group is generally a negative factor with respect to the bioavailability. The compounds of the invention include those having an amidino group of which the free amino group has been replaced with a tertiary amine. These compounds have enhanced hydrophobic nature than those compounds having an unsubstituted amidino group, and thus

they acquire still higher bioavailability.

As regards the preparation, the β-amino acid residues of the compounds of the present invention can be synthesized by directly applying the methods for synthesizing β-lactams which are used as antiboitics. Since a wide variety of methods for synthesizing β-lactams have been developed, the compounds of the present invention can be synthesized easily and at low cost.

As is clear from the description, the novel compounds of the present invention having substituted β-amino acid residues are useful as fibrinogen receptor antagonists.

The present invention will now be explained in greater detail with reference to the following examples.

[Synthesis of Compounds]

[Example 1]

Ethyl N-(N-(4-(4-morpholinoimidoyl)benzoyl)-β-ethyl-α,α-dimethyl-β-alanyl)-4-piperidineacetate

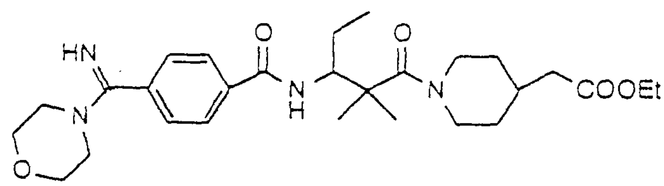

β-Ethyl-α,α-dimethyl-β-alanine hydrochloride (2.0 g, 11.07 mmol) was dissolved in a 10% aqueous sodium carbonate solution (11.7ml). To the resultant solution was added di-t-buthyldicarbonate (2.9 g, 13.21 mmol) in dioxane (40 ml) under ice-cooling. The mixture was stirred overnight at room temperature and the solvent was distilled off therefrom. The residue was dissolved in water and then washed with ether to separate an aqueous layer. Thereafter, the aqueous layer was adjusted to pH 3 with citric acid and then extracted with ethyl acetate. The ethyl acetate layer collected was washed with saturated aqueous NaCl solution, dried over anhydrous sodium sulfate and evaporated in vacuo to remove the solvent. The residue was reprecipitated from ether/hexane to give N-BOC-β-ethyl-α,α-dimethyl-β-alanine (2.7 g, quant.) in a crystalline form.

The crystalline product (1.2 g, 4.89 mmol) thus obtained was dissolved in methylene chloride (50 ml). To the resultant solution were added HATU regient (2.8 g, 7.37 mmol) and diisopropylethylamine (5.2 ml, 29.35 mmol). The mixture was stirred for 30 min and ethyl 4-piperidinyl acetate (1.26 g, 7.34 mmol) was dissolved thereto, followed by stirring overnight. After distilling off the solvent from the reaction solution, the residue was dissolved in ethyl acetate, washed with a 5% aqueous citric acid solution, a 5% aqueous sodium bicarbonate solution and a saturated aqueous sodium chloride solution, respectively, each three times and then dried over anhydrous sodium sulfate. After distilling off the solvent, the residue was applied to a silica gel column (2.2 x 15 cm) and eluted with hexane:ethyl acetate (5:1). The desired fractions were collected and the solvent was distilled off therefrom to give ethyl N-(N-BOC-β-ethyl-α,α-dimethyl-β-alanyl)-4-piperidineacetate (1.77 g, 90.8%) in a powdery form.
MS: [M+H]+ calcurated 399.286, found 399.5.

To the above-obtained ethyl N-(N-BOC-β-ethyl-α,α-dimethyl-β-alanyl)-4-piperidineacetate (0.38 g, 0.95 mmol) were added anisole (0.3 ml) and TFA (10 ml). The resultant mixture was stirred for one hour under ice-cooling. After distilling off the TFA from the reaction mixture at room temperature, the residue was washed with hexane three times and then dissolved in DMF (20 ml) under ice-cooling. After neutralizing the resultant solution with triethylamine, 4-(4-morpholinoimidoly) benzoate (0.25 g, 1.05 mmol), HOBT (0.14 g, 1.05 mmol) and WSDC (0.22 g, 1.14 mmol) were added to the solution and then stirred overnight. After distilling off the solvent from the solution, the residue was dissolved in ethyl acetate. The resultant solution was washed with a 5% aqueous citric acid solution, a 5% aqueous sodium bicarbonate solution and a saturated aqueous sodium chloride solution, respectively, each three times and then dried over anhydrous sodium sulfate. After distilling off the solvent from the solution, the residue was applied to a silica gel column (ϕ2.1 x 20 cm) and eluted with chloroform:ethanol (30:1). The desired fractions were collected and the solvent was distilled off therefrom to give the titled compound (108 mg, 22%) in a powder form.
NMR: $^1$H (270MHz: $CDCl_3$: 25°C) 0.92, t, J=7.3Hz, 3H: 1.11-1.24, m, 2H: 1.26, t, J=6.8Hz, 3H: 1.31, s, 3H: 1.38, s, 3H: 1.62-1.88, m, 4H: 1.95-2.15, m, 1H: 2.25, d, J=7.3Hz, 2H: 2.68-2.95, m, 2H: 3.33-3.45, m, 2H: 3.64-3.75, m, 2H: 3.83-4.08, m, 5H, 4.13, q, J=7.3Hz, 2H: 4.40, br-d, J=12.7hz, 2H: 7.53, d, J=7.8Hz, 2H: 7.90, d,J=7.8Hz, 2H
$^{13}$C (67.5MHz:$CDCl_3$:25°C) 11.62, 14.22, 23.80, 24.13, 24.27, 31.96, 32.20, 33.10, 40.80, 40.99, 45.37, 46.49, 47.34,

50.12, 60.39, 61.03, 65.79, 66.32, 128.00, 128.37, 130.76, 138.85, 164.55, 166.19, 172.19, 175.14
MS: $[M+H]^+$ calculated: 515.323, found: 515.5
HRMS: $C_{28}H_{43}N_4O_5$ $[M+H]^+$ calculated: 515.3233, found: 515.3257

[Example 2]

N-(N-(9-(4-morpholinoimidoyl)benzoyl)-β-ethyl-α,α-dimethyl-β-alanyl)-4-piperidineacetic acid

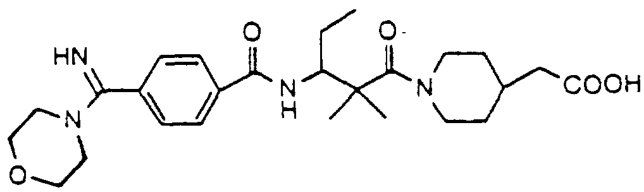

Ethyl N-(N-(4-(4-morpholinoimidoyl)benzoyl)-β-ethyl-α,α-dimethyl-β-alanyl)-4-piperidineacetate (28 mg, 54.4 mmol), a product of Example 1, was dissolved in ethanol (10 ml) and a 4N aqueous NaOH solution (3 ml) was added thereto under ice-cooling, followed by stirring for three hours. The resultant solution was neutralized with acetic acid under ice-cooling. After destilling off the ethanol from the neutralized solution, the residue was dissolved in water. The resultant solution was subjected to high performance liquid chromatography (HPLC) [column: ODS 5C18 (μ bondasphere, ϕ19 x 150 mm); mobile phase: (A) 0.1% aqueous TFA solution, (B) 100% $CH_3CN$/0.1% TFA; gradient: (A):(B) =76:24 - 74:26; 6 min; flow rate: 17 ml/min]. The desired fractions were collected and lyophilized to give the titled compound (20.3 mg, 77%). NMR: $^1H$ (400MHz: $CD_3OD$: 25°C) 0.93, t, J=7.2Hz, 3H: 1.13-1.35, m, 2H: 1.27, s, 3H: 1.30, s, 3H: 1.51-1.68, m, 2H: 1.84, br-t, J=13.6Hz, 2H: 1.98-2.12, m, 1H: 2.25, d, J=6.8Hz, 2H: 2.60-3.10, m, 2H: 3.45, t, J=4.4Hz, 2H: 3.73, t, J=4.8Hz, 2H: 3.81, t, J=4.4Hz, 2H: 3.92, 2H: 3.92, t, J=4.4Hz, 2H: 4.46, dd, J=10.4Hz, 4.0Hz, 1H: 4.54, br-d, J=13.6Hz, 2H: 7.72, d, J=8.4Hz, 2H: 8.02, d, J=8.4Hz, 2H
$^{13}C$ (100MHz:$CD_3OD$:25°C) 12.73, 24.16, 24.60, 25.00, 34.02, 34.16, 35.17, 42.34, 48.00, 48.89, 52.20, 59.24, 67.12, 68.13, 130.29, 130.52, 133.58, 140.66, 166.97, 170.37, 176.81, 177.10
MS: $[M+H]^+$ calculated: 487.292, found: 487.4
HRMS: $C_{26}H_{39}N_4O_5$ $[M+H]^+$ calculated: 487.2920, found: 487.2897

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 18.63 min was obtained.

[Example 3]

Ethyl N-(N-(4-(4-morpholinoimidoyl)-2-fluorobenzoyl)-β-ethyl-α,α-dimethyl-β-alanyl)-4-piperidineacetate

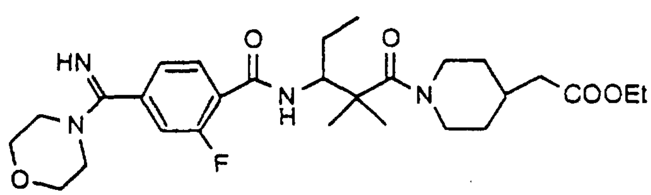

To methyl N-BOC-β-ethyl-α,α-dimethyl-β-alanyl-4-piperidineacetate (1.0 g, 2.51 mmol), which was described in Example 1, were added anisole (1.0 ml) and TFA (30 ml) and the mixture was stirred for one hour under ice-cooling.

After distillation off the TFA from the mixture at room temperature, the residue was washed with hexane three times and then dissolved in DMF (50 ml) under ice-cooling. The resultant solution was neutralized with triethylamine and then 2-fluoro-4-cyanobenzoic acid (0.46 g, 2.76 mmol), HOBT (0.37 g, 2.76 mmol) and WSDC (0.63 g, 3.26 mmol) were added thereto, followed by stirring overnight. After distillation off the solvent from the solution, the residue was dissolved in ethyl acetate and washed with a 5% aqueous citric acid solution, a 5% aqueous sodium bicarbonate solution and a saturated aqueous sodium chloride solution, respectively, each three times and then dried over anhydrous sodium sulfate. After distillation off the solvent, the residue was applied to a silica gel column (2.2 x 15 cm) and eluted with hexane:ethyl acetate (3:1). The desired fractions were collected and the solvent was distilled off therefrom to give ethyl N-(N-2-fluoro-4-cyanobenzoyl-β-ethyl-α,α-dimethyl-β-alanyl-4-piperidineacetate (855 mg, 76.5%) in a powder form.
MS: $[M+Na]^+$ calculated 446.246, found 446.5

Ethyl N-(N-4-cyano-2-fluorobenzoyl)-β-ethyl-α,α-dimethyl-β-alanyl)-4-piperidineacetate (4.1 g, 9.8 mmol) thus obtained was dissolved in pyridine (200 ml). After addition of triethylamine (50 ml), the reaction solution was saturated with hydrogen sulfide, stoppered and then stirred at room temperature overnight. After distilling off the pyridine from the resultant, the residue was subjected to azeotropic distillation with toluene twice and then dissolved in acetone (200 ml). To the resultant solution was added methyl iodide (20 ml) and the mixture was refluxed for 30 min. After distilling off the solvent from the reaction solution, the residue was dissolved in methanol (200 ml) and then morpholine (20 ml) was added thereto, followed by reflux for two hours. After distilling off the solvent from the reaction solution, the residue was dissolved in chloroform, washed with a saturated aqueous sodium chloride solution and dried over anhydrous sodium sulfate. After distilling off the solvent, the residue was applied to a silica gel column (1.5 x 14 cm) and eluted with chloroform:methanol (30:1). The desired fractions was collected and the solvent was distilled off therefrom to give the titled compound (1.4 g, 28%) in a powder form.
NMR: $^1H$ (270MHz: $CDCl_3$: 25°C) 0.93, m, 3H: 1.12-1.41, m, 8H: 1.26, t, J=7.0Hz, 3H: 1.57-1.89, m, 4H: 1.92-2.16, m, 1H: 2.24, d, J=6.8Hz, 2H: 2.82, m, 2H: 3.41, br-s, 2H, 3.70, br-s, 2H: 3.85-3.97, m, 4H: 4.03-4.19, m, 1H: 4.13, q, J=7.3Hz, 2H: 4.27-4.47, m, 2H: 7.32-7.43, m, 2H: 7.68-7.82, m, 1H: 8.00, t, J=7.6Hz, 1H
$^{13}C$ (67.5MHz:$CDCl_3$:25°C) 11.56, 14.22, 23.83, 23.93, 24.09, 31.97, 32.15, 32.96, 33.10, 40.60, 40.82, 40.07, 45.07 (br), 46.46, 47.24, 50.11, 60.42, 60.72, 65.59, 66.20, 77.21, 116.41, 11.80, 124.22, 126.39, 126.58, 132.05, 132.18, 132.65, 157.86, 161.59, 162.74, 163.06, 172.26, 174.81
MS: $[M+H]^+$ calculated: 533.314, found: 533.4
HRMS: $C_{28}H_{42}FN_4O_5$ $[M+H]^+$ calculated: 533.3139, found: 533.3136

[Example 4]

N-{N-(4-(4-morpholinoimidoyl)-2-fluorobenzoyl)-β-ethyl-α,α-dimethyl-β-alanyl)-4-piperidineacetic acid

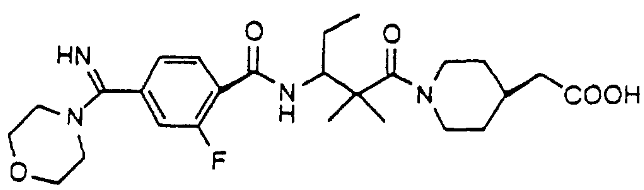

The same procedure as in Example 2 was performed with ethyl N- (N-(4-(4-morpholinoimidoyl)-2-fluorobenzoyl)-β-ethyl-α,α-dimethyl-β-analyl)-4-piperidineacetate (200 mg, 0.38 mmol) produced in Example 3 to give the titled compound (160 mg, 84%).
NMR: $^1H$ (400MHz: $CD_3OD$: 25°C) 0.97, t, J=7.6Hz, 3H: 1.12-1.25, m, 2H: 1.26, s, 3H: 1.34, s, 3H: 1.50-1.63, m, 2H: 1.78-1.91, m, 2H: 2.06, br-s, 1H: 2.26, d, J=7.2Hz, 2H: 2.78-3.08, m, 2H: 3.39-3.50, m, 2H: 3.65-3.76, m, 2H: 3.76-3.84, m, 2H: 3.86-3.95, m, 2H: 4.37-4.45, m, 1H: 4.52, br-d, J=12.4Hz, 2H: 7.53, d, J=7.6Hz, 1H: 7.58, d, J=10.4Hz: 7.85, t, J=7.6Hz, 1H:
$^{13}C$ (100MHz:$CD_3OD$:25°C) 12.68, 24.14, 24.71, 25.28, 34.04, 34.13, 35.15, 42.34, 47.30, 47.75, 48.92, 49.29, 52.20, 59.93, 67.05, 68.03, 118.47, 118.73, 126.45, 129.77, 129.92, 133.45, 134.51, 134.58, 160.35, 162.86, 165.60, 167.01, 176.77, 177.05
MS: $[M+H]^+$ calculated: 505.283, found: 505.5
HRMS: $C_{26}H_{38}FN_4O_5$ $[M+H]^+$ calculated: 505.2826, found: 505.2827

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 19.46 min was obtained

[Example 5]

Ethyl N-(N-(4-(N,N-diethylaminoimidoyl)-2-fluorobenzoyl)-β-ethyl-α,α-dimethyl-β-alanyl)-4-piperidineacetate

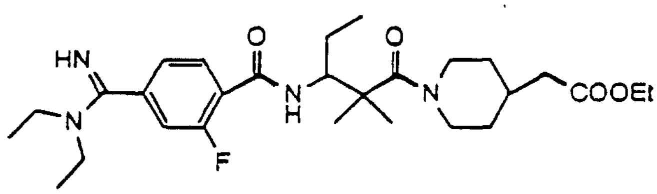

The same procedure as in Example 3 was performed with ethyl N-(N-(4-cyano-2-fluorobenzoyl)-β-ethyl-α,α-dimethyl-β-analyl)-4-piperidineacetate (1.4 g, 3.1 mmol) using diethylamine (3.2 ml) as the amine component to give the titled compound (224 mg, 15%).
NMR: $^{1}$H (270MHz: $CDCl_3$: 25°C) 0.94, t, J=7.4Hz, 3H: 1.17, t, J=7.0Hz, 3H: 1.26, t, J=7.0Hz, 3H: 1.12-1.39, m, 11H: 1.55-1.76, m, 2H: 1.80, br-d, J=11.9Hz, 1.96-2.15, m, 1H: 2.25, d, J=6.8Hz, 2.72-2.91, m, 2H: 3.29, q, J=6.1Hz, 2H: 3.74, q, J=6.8Hz, 2H: 4.13, q, J=J=7.3Hz, 2H: 4.39-4.18, m, 1H: 4.42, br-d, J=12.7Hz, 2H: 7.27, br-t, J=8.6Hz, 7.61, br-t, J=8.5Hz, 1H: 7.98, t, J=7.6Hz, 1H
$^{13}$C (67.5MHz:$CDCl_3$:25°C) 11.17, 11.48, 13.62, 14.18, 23.64, 23.83, 23.93, 31.97, 32.12, 33.10, 40.82, 43.55, 45.02, 45.37, 46.53, 46.85, 60.22, 60.35, 76.53, 77.00, 77.21, 77.47, 115.48, 115.87, 123.28, 123.33, 125.76, 125.96, 132.50, 132.97, 133.11, 157.80, 161.51, 162.84, 172.21, 174.70
MS: $[M+H]^+$ calculated: 519.335, found: 519.5
HRMS: $C_{28}H_{44}FN_4O_4$ $[M+H]^+$ calculated: 519.3346, found: 519.3360

[Example 6]

N-(N-(4-(N,N-diethylaminoimidoyl)-2-fluorobenzoyl)-β-ethyl-α,α-dimethyl-β-alanyl)-4-piperidineacetic acid

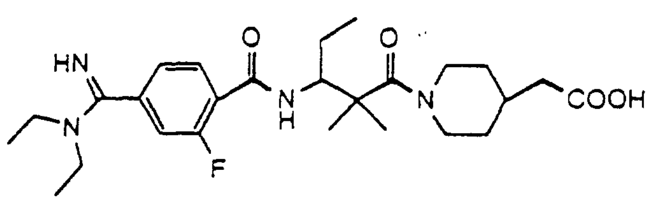

The same procedure as in Example 2 was performed with ethyl N-(N-(4-(N,N-diethylaminoimidoyl)-2-fluorobenzoyl)-β-ethyl-α,α-dimethyl-β-alanyl)-4-piperidineacetate (56 mg, 0.11 mmol) preparead in Example 5 to give the titled compound (29 mg, 54.7%).
NMR: $^{1}$H (400MHz: $CD_3OD$: 25°C) 0.97, t, J=7.2Hz, 3H: 1.13-1.27, m, 2H: 1.19, t, J=7.2Hz, 3H: 1.26, s, 3H: 1.34, s, 3H: 1.38, t, J=7.2Hz, 3H: 1.50-1.63, m, 2H: 1.77-1.92, m, 2H: 1.98-2.13, m, 1H: 2.25, d, J=6.8Hz, 2H: 2.60-3.05, m, 2H: 3.34, q, J=7.6Hz, 2H: 3.69, q, J=7.6Hz, 2H: 4.37-4.45, m, 1H: 4.52, br-d, J=12.8Hz, 2H: 7.49, dd, J=7.6Hz, 1.2Hz, 1H:7.57, d, J=10.4Hz, 1H: 7.84, t, J=6.8Hz, 1H
$^{13}$C (100MHz:$CD_3OD$:25°C) 12.16, 12.68, 14.52, 24.14, 24.73, 25.28, 34.05, 34.15, 35.19, 42.45, 45.45, 47.37, 47.77, 59.91, 117.74, 118.00, 125.70, 129.32, 129.46, 133.38, 135.35, 165.40, 167.08, 168.20, 176.88, 177.07

MS: $[M+H]^+$ calculated: 491.303, found: 491.4
HRMS: $C_{26}H_{40}FN_4O_4$ $[M+H]^+$ calculated: 491.3033, found: 491.3013

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min) As a result, a spectrum having a single peak at a retention time of 21.19 min was obtained.

[Example 7]

Ethyl N-(N-(4-(1-piperidinoimidoyl)-2-fluorobenzoyl)-β-ethyl-α,α-dimethyl-β-alanyl)-4-piperidineacetate

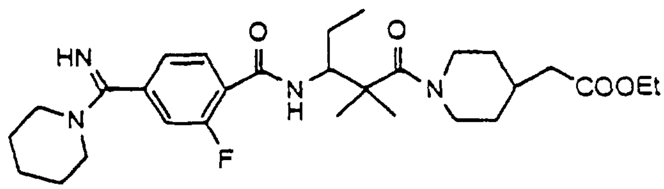

The same procedure as in Example 3 was performed with ethyl N-(N-(4-cyano-2-fluorobenzoyl)-β-ethyl-α,α-dimethyl-β-alanyl)-4-piperidineacetate (1.4 g, 3.1 mmol) using piperidine (3.1 ml) as the amine component to give the titled compound (487 mg, 30%).
NMR: $^1H$ (270MHz: $CDCl_3$: 25°C) 0.95, t, J=7.3Hz, 3H: 1.09-1.46, m, 2H: 1.26, t, J=7.2Hz, 3H: 1.34, s, 3H: 1.39, s, 3H: 1.59-1.96, m, 8H: 1.96-2.19, m, 1H: 2.23-2.30, m, 2H: 2.74-2.92, m, 2H: 3.39, br-t, J=5.4Hz, 2H: 3.79-3.86, m, 2H: 4.02- 4.19, m, 1H: 4.14, q, J=7.0Hz, 2H: 4.40, br-d, J=12.2Hz, 2H: 7.22-7.37, m, 2H: 8.06, t, J=7.6Hz, 1H
$^{13}C$ (67.5MHz:$CDCl_3$:25°C) 11.56, 14.23, 20.80, 23.14, 23.83, 24.03, 24.15, 24.93, 26.24, 31.94, 32.17, 33.07, 40.79, 45.16, 45.40, 46.46, 48.73, 51.71, 60.48, 61.26, 77.20, 115.89, 116.29, 123.76, 126.31, 126.51, 132.79, 133.06, 133.11, 162.14, 162.64, 172.27, 174.78, 175.10
MS: $[M+H]^+$ calculated: 531.335, found: 531.3
HRMS: $C_{29}H_{44}FN_4O_4$ $[M+H]^+$ calculated: 531.3346, found: 531.3352

[Example 8]

N-(N-(4-(1-piperidinoimidoyl)-2-fluorobenzoyl)-β-ethyl-α,α-dimethyl-β-alanyl)-4-piperidineacetic acid

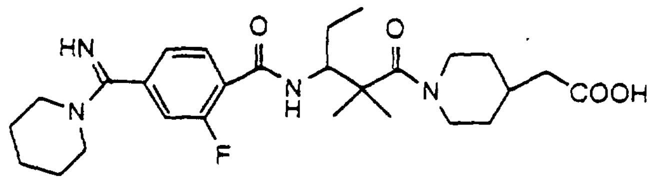

The same procedure as in Example 2 was performed with ethyl N-(N-(4-(1-piperidinoimidoyl)-2-fluorobenzoyl)-β-ethyl-α,α-dimethyl-β-alanyl)-4-piperidineacetate (100 mg, 0.188 mmol) prepared in Example 7 to give the titled compound (85.8 mg, 91%).
NMR: $^1H$ (400MHz: $CD_3OD$: 25°C) 0.97, t, J=7.2Hz, 3H: 1.13-1.25, m, 2H: 1.26, s, 3H: 1.34, s, 3H: 1.48-1.71, m, 4H: 1.72-1.94, m, 6H: 2.06, br-s, 1H: 2.26, d, J=6.4Hz, 2H: 2.65-3.10, m, 2H: 3.40, br-s, 2H: 3.76, br-s, 2H: 4.37-4.46, m, 1H: 4.53, br-d, J=12Hz, 2H: 7.50, d, J=7.6Hz, 1H: 7.56, d, J=9.6Hz, 1H: 7.84, t, J=6.8Hz, 1H
$^{13}C$ (100MHz:$CD_3OD$:25°C) 12.68, 24.03, 24.65, 25.08, 25.20, 26.83, 27.84, 34.02, 34.15, 35.15, 42.34, 47.31, 47.73, 53.44, 59.62, 118.11, 118.36, 126.08, 129.76, 133.30, 135.24, 135.32, 162.82, 164.54, 167.19, 176.81, 176.98

MS: $[M+H]^+$ calculated: 503.303, found: 503.3
HRMS: $C_{27}H_{40}FN_4O_4$ $[M+H]^+$ calculated: 503.3033, found: 503.3048

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 22.14 min was obtained.

[Example 9]

Ethyl N-(N-(4-1-pyrrolidinoimidoyl)-2-fluorobenzoyl)-β-ethyl-α,α-dimethyl-β-alanyl)-4-piperidineacetate

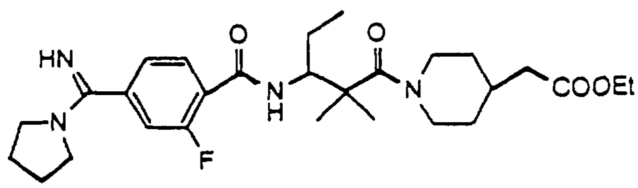

The same procedure as in Example 3 was performed with ethyl N-(N-(4-cyano-2-fluorobenzoyl)-β-ethyl-α,α-dimethyl-β-alanyl)-4-piperidineacetate (1.4 g, 3.1 mmol) using pyrrolidine (2.6 ml) as the amine component to give the titled compound (398 mg, 25%).
NMR: $^1H$ (270MHz: $CDCl_3$: 25°C) 1.16-1.42, m, 2H: 1.26, t, J=7.3Hz, 6H: 1.31, s, 3H: 1.37, s, 3H: 1.59-1.89, m, 4H: 1.91-2.30, m, 5H: 2.25, d, J=7.3Hz, 2H: 2.71-2.91, m, 2H: 3.43, br-t, J=6.3Hz, 2H: 3.76, br-s, 2H: 4.06-4.48, m, 1H: 4.13, q, J=6.9Hz, 2H: 4.41, br-d, J=12.7Hz, 2H: 7.28-7.43, m, 2H: 7.66, br-d, J=8.2Hz, 1H: 8.00, br-t, J=8.2Hz, 1H
$^{13}C$ (67.5MHz:$CDCl_3$:25°C) 11.54, 14.20, 23.81, 23.86, 24.06, 24.85, 24.96, 25.51, 29.66, 31.99, 32.17, 32.29, 33.12, 40.83, 45.05, 45.37, 46.47, 49.13, 52.12, 60.39, 60.67, 77.20, 115.70, 116.10, 12.62, 125.94, 126.13, 132.60, 133.23, 133.35, 157.82, 160.89, 161.02, 161.53, 162.74, 172.24, 174.79
MS: $[M+H]^+$ calculated: 517.319, found: 517.4
HRMS: $C_{28}H_{42}FN_4O_4$ $[M+H]^+$ calculated: 517.3189, found: 517.3207

[Example 10]

N-(N-(4-(1-pyrrolidinoimidoyl)-2-fluorobenzoyl)-β-ethyl-α,α-dimethyl-β-alanyl)-4-piperidineacetic acid

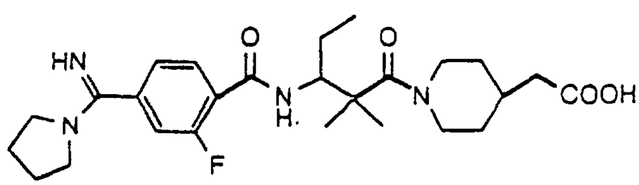

The same procedure as in Example 2 was performed with ethyl N-(N-(4-(1-pyrrolidinoimidoyl)-2-fluorobenzoyl)-β-ethyl-α,α-dimethyl-β-alanyl)-4-piperidineacetate (100 mg, 0.194 mmol) prepared in Example 9 to give the titled compound (36.4 mg, 38.5%).
NMR: $^1H$ (400MHz: $CD_3OD$: 25°C) 0.97, t, J=7.2Hz, 3H: 1.14-1.28, m, 2H: 1.26, s, 3H: 1.34, s, 3H: 1.50-1.62, m, 2H: 1.78-1.91, m, 2H: 1.94-2.13, m, 3H: 2.13-2.3, m, 2H: 2.26, d, J=7.2Hz, 2H: 3.48, t, J=6.8Hz, 2H: 3.63, t, J=7.2Hz, 2H: 4.38-4.46, m, 1H: 4.53, br-d, J=12.8Hz, 2H: 7.53, d, J=8.0Hz, 1H: 7.57, d, J=10.4Hz, 1H: 7.83, t, J=6.8Hz, 1H
$^{13}C$ (100MHz:$CD_3OD$:25°C) 12.68, 24.09, 24.69, 25.24, 26.68, 27.29, 34.05, 34.15, 35.17, 42.34, 47.39, 47.75, 50.93, 54.03, 59.77, 117.81, 118.07, 125.86, 129.50, 129.66, 133.25, 135.70, 135.79, 160.22, 163.28, 167.17, 167.21, 176.79,

177.03
MS: $[M+H]^+$ calculated: 489.288, found: 489.1
HRMS: $C_{26}H_{39}FN_4O_4$ $[M+H]^+$ calculated: 489.2876, found: 489.2862

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 20.70 min was obtained.

[Example 11]

Ethyl N-(N-(4-(1-4-piperidonoimidoyl)-2-fluorobenzoyl)-β-ethyl-α,α-dimethyl-β-alanyl)-4-piperidineacetate

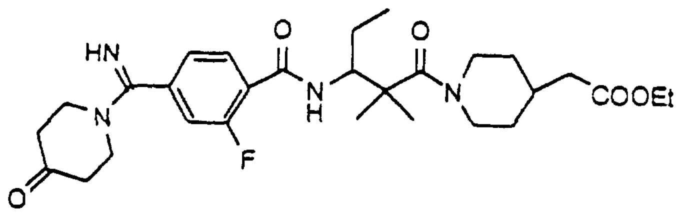

The same procedure as in Example 3 was performed with ethyl N-(N-(4-cyano-2-fluorobenzoy)-β-ethyl-α,α-dimethyl-β-alanyl)-4-piperidineacetate (1.4 g, 3.1 mmol) using an amine which had been prepared by dissolving 4-piperidone monohydrate hydrochloride (4.8 g) in methanol and then neutralizing the resultant with triethylamine to give the titled compound (183 mg, 11%).
NMR: $^1H$ (270MHz: $CDCl_3$: 25°C) 0.93, t, J=7.0Hz, 3H: 1.09-1.43, m, 2H: 1.26, t, J=7.0Hz, 3H: 1.31, s, 3H: 1.36, s, 3H: 1.59-1.84, m, 2H: 1.80, br-d, J=10.8Hz, 2H: 1.96-2.15, m, 1H: 2.25, d, J=6.8Hz, 2H: 2.57, m, 2H: 2.82, br-s, 4H: 3.75, m, 2H: 4.02-4.19, m, 4H: 4.13, q, J=7.1Hz, 2H: 4.37, br-d, J=12.2Hz, 2H: 7.43, br-d, J=8.6Hz, 2H: 7.70, br-t, J=8.2Hz, 1H: 7.96, br-t, J=7.0Hz, 1H
$^{13}C$ (67.5MHz:$CDCl_3$:25°C) 11.53, 14.20, 23.77, 23.89, 24.09, 31.94, 32.14, 33.09, 38.08, 39.46, 40.80, 44.79, 45.00, 45.42, 46.41, 47.34, 60.39, 60.82, 77.20, 116.36, 116.76, 124.20, 126.54, 12.74, 132.24, 132.36, 157.72, 161.44, 162.81, 163.61, 172.21, 174.82,
MS: $[M+H]^+$ calculated: 545.314, found: 545.3
HRMS: $C_{29}H_{42}FN_4O_5$ $[M+H]^+$ calculated: 545.3139, found: 545.3147

[Example 12]

N-(N-(4-(1-4-piperidonoimidoyl)-2-fluorobenzoyl)-β-ethyl-α,α-dimethyl-β-alanyl)-4-piperidineacetic acid

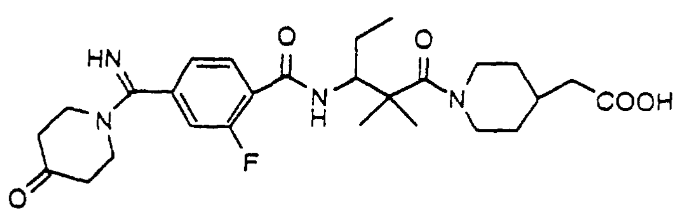

The same procedure as in Example 2 was performed with ethyl N-(N-(4-(1-4-piperidonoimidoyl)-2-fluorobenzoyl)-β-ethyl-α,α-dimethyl-β-alanyl)-4-piperidineacetate (30 mg, 0.06 mmol) prepared in Example 11 to give the titled

compound (15 mg, 53%).
NMR: $^1$H (400MHz: $CD_3OD$: 25°C) 0.97, t, J=7.2Hz, 3H: 1.13-1.28, m, 2H: 1.26, s, 3H: 1.34, s, 3H: 1.50-1.63, m, 2H: 1.75-1.90, m, 4H: 1.94-2.12, m, 3H: 2.25, d, J=6.8Hz, 2H: 2.68-3.10, m, 2H: 3.40-3.50, m, 2H: 3.74-3.85, m, 2H: 4.36-4.45, m, 1H: 4.52, br-d, J=12.8Hz, 2H: 7.52, d, J=8.0Hz, 1H: 7.58, d, J=9.6Hz, 1H: 7.84, t, J=7.2Hz, 1H
$^{13}C$ (100MHz:$CD_3OD$:25°C) 12.68, 24.12, 24.71, 25.26, 34.05, 34.15, 35.17, 36.27, 37.11, 42.39, 46.40, 47.35, 47.79, 59.88, 96.09, 118.22, 118.49, 126.21, 129.66, 129.83, 133.40, 135.13, 135.21, 160.33, 162.84, 165.05, 167.08, 176.85, 177.05
MS: $[M+H]^+$ calculated: 517.283, found: 517.2 (for monohydrate: 535.3)
HRMS: $C_{27}H_{38}FN_4O_5$ $[M+H]^+$ calculated: 517.2826, found: 517.2823

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 18.49 min was obtained.

[Example 13]

Ethyl N-(N-(4-(4-hydroxy-1-piperidinoimidoyl)-2-fluorobenzoyl)-β-ethyl-α,α-dimethyl-β-alanyl)-4-piperidineacetate

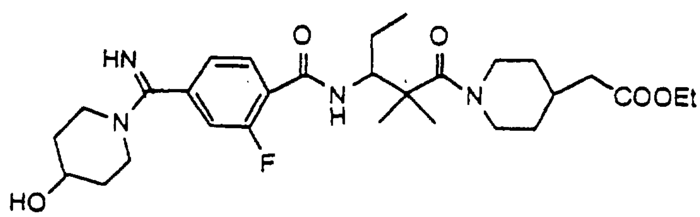

The same procedure as in Example 3 was performed with ethyl N-(N-(4-cyano-2-fluorobenzoyl)-β-ethyl-α,α-dimethyl-β-alanyl)-4-piperidineacetate (1.4 g, 3.1 mmol) using 4-hydroxypiperidine (3.1 g) as the amine component to give the titled compound (440 mg, 26%).
NMR: $^1$H (270MHz: $CDCl_3$: 25°C) 0.92, t, J=7.3Hz, 3H: 1.09-1.39, m, 2H: 1.25, t, J=7.0Hz, 6H: 1.29, s, 3H: 1.35, s, 3H: 1.55-1.92, m, 7H: 1.92-2.13, m, 2H: 2.24, d, J=6.8Hz, 2H: 2.74-2.93, m, 2H: 3.22-3.36, m, 1H: 3.53-3.68, m, 1H: 3.68-3.83, m, 1H: 3.94-4.19, m, 3H: 4.12, q, J=7.2Hz, 2H: 4.37, br-d, J=11.6Hz, 2H: 7.29-7.39, m, 2H: 7.72, br-t, J=8.1Hz, 1H: 7.94, t, J=7.6Hz, 1H
$^{13}C$ (67.5MHz:$CDCl_3$:25°C) 11.50, 14.18, 23.77, 23.98, 31.94, 32.09, 32.46, 33.07, 33.39, 40.79, 43.83, 45.03, 45.45, 46.47, 47.30, 60.42, 60.64, 63.91, 77.20, 113.76, 116.09, 116.48, 118.07, 123.97, 126.14, 126.34, 132.41, 132.74, 132.88, 157.80, 160.29, 160.84, 161.53, 162.26, 163.03, 172.29, 174.90
MS: $[M+H]^+$ calculated: 547.330, found: 547.4
HRMS: $C_{29}H_{44}FN_4O_5$ $[M+H]^+$ calculated: 547.3295, found: 547.3309

[Example 14]

N-(N-(4-(4-hydroxy-1-piperidinoimidoyl)-2-fluorobenzoyl)-β-ethyl-α,α-dimethyl-β-alanyl)-4-piperidineacetic acid

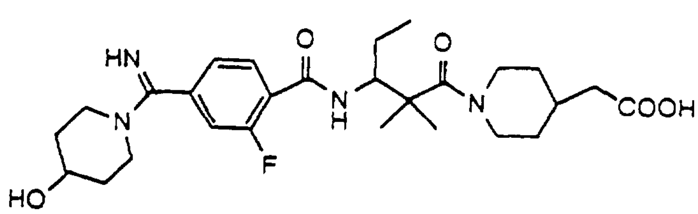

The same procedure as in Example 2 was performed with ethyl N-(N-(4-(4-hydroxy-1-piperidineimidoyl)-2-fluorobenzoyl)-β-ethyl-α,α-dimethyl-β-alanyl)-4-piperidineacetate (200 mg, 0.37 mmol) prepared in Example 13 to give the titled compound (162 mg, 86%).
NMR: $^{1}H$ (400MHz: $CD_3OD$: 25°C) 0.91-1.04, m, 3H: 1.12-1.28, m, 2H: 1.26, s, 3H: 1.34, s, 3H: 1.50-1.67, m, 3H: 1.74-1.95, m, 4H: 2.09, br-s, 2H: 2.26, br-d, J=6.4Hz, 2H: 2.70-3.10, m, 2H: 3.30-3.38, br-s, 1H: 3.55-3.74, m, 2H: 3.90-4.08, m, 2H: 4.42, br-s, 1H: 4.53, br-d, J=12.0Hz, 2H: 7.51, br-d, J=7.6Hz, 1H:7.57, br-d, J=10.0Hz, 1H:7.84, m, 1H
$^{13}C$ (100MHz:$CD_3OD$:25°C) 12.68, 24.03, 24.65, 25.20, 34.02, 34.15, 34.31, 35.15, 35.35, 42.32, 45.67, 47.37, 47.68, 59.66, 66.08, 118.14, 118.40, 126.12, 129.66, 129.83, 133.34, 135.13, 135.21, 160.30, 162.80, 164.81, 167.17, 176.79, 176.98
MS: $[M+H]^+$ calculated: 519.298, found: 519.3
HRMS: $C_{27}H_{40}FN_4O_5$ $[M+H]^+$ calculated: 519.2982, found: 519.2981

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 18.62 min was obtained.

[Example 15]

Ethyl N-(N-(4-(3-thiazolidinoimidoyl)-2-fluorobenzoyl)-β-ethyl-α,α-dimethyl-β-alanyl)-4-piperidineacetate

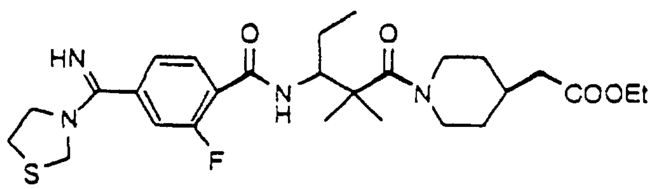

The same procedure as in Example 3 was performed with ethyl N-(N-(4-cyano-2-fluorobenzoyl)-β-ethyl-α,α-dimethyl-β-alanyl)-4-piperidineacetate (1.48 g, 3.3 mmol) using thiazolidine (2.5 ml) as the amine component to give the titled compound (314.mg, 23%).
NMR: $^{1}H$ (400MHz: $CDCl_3$: 25°C) 0.92, t, J=7.2Hz, 3H: 1.16, br-q, J=12.0Hz, 2H: 1.24, t, J=7.2Hz, 3H: 1.30, s, 3H: 1.35, s, 3H: 1.59-1.73, m, 2H: 1.78, br-d, J=12.0Hz, 2H: 1.97-2.09, m, 1H: 2.23, d, J=6.8Hz, 2H: 2.70-2.93, m, 2H: 3.05, t, J=6.4Hz, 1H: 3.31, t, J=6.0Hz, 1H: 3.72, t, J=5.6Hz, 1H: 4.02-4.08, m, 2H: 4.12, q, J=6.8Hz, 2H: 4.37, br-d, J=13.2Hz, 2H: 4.41, s, 1H: 4.79, s, 1H: 7.34, t, J=8.4Hz, 1H: 7.38, t, J=8.0Hz, 1H: 7.98, q, J=8.0Hz, 1H
$^{13}C$ (100MHz:$CDCl_3$:25°C) 11.65, 14.34, 23.99, 24.10, 24.23, 30.23, 30.54, 32.11, 32.29, 33.25, 40.95, 45.24, 45.55, 46.65, 51.06, 51.79, 54.42, 55.23, 60.51, 61.06, 61.14, 114.57, 115.85, 115.98, 116.13, 116.26, 117.44, 123.61, 123.70, 123.73, 126.83, 126.95, 132.79, 132.88, 133.03, 158.63, 160.73, 161.12, 161.32, 161.61, 162.71, 162.80, 172.31, 175.11
MS: $[M+H]^+$ calculated: 535.275, found: 535.4
HRMS: $C_{27}H_{40}FN_4O_4S$ $[M+H]^+$ calculated: 535.2754, found: 535.2750

[Example 16]

N-(N-(4-(3-thizolidinoimidoyl)-2-fluorobenzoyl)-β-ethyl-α,α-dimethyl-β-alanyl)-4-piperidineacetic acid

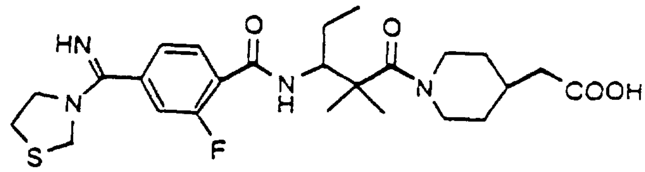

The same procedure as in Example 2 was performed with ethyl N-(N-(4-(3-thiazolydinoimidoyl)-2-fluorobenzoyl)-β-ethyl-α,α-dimethyl-β-alanyl)-4-piperidineacetate (150 mg, 0.28 mmol) prepared in Example 15 to give the titled compound (101 mg, 71%).
NMR: $^1$H (400MHz: $CD_3OD$: 25°C) 0.97, t, J=7.6Hz, 3H: 1.12-1.28, m, 2H: 1.26, s, 3H: 1.34, s, 3H: 1.49-1.62, m, 2H: 1.84, br-t, J=11.2Hz, 2H: 1.98-2.12, m, 1H: 2.26, d, J=7.6Hz, 2H: 2.69-3.08, m, 2H: 3.13, t, J=6.4Hz, 1H: 3.38, t, J=6.4Hz, 1H: 3.79, t, J=6.0Hz, 1H: 3.92, t, J=6.0Hz, 1H: 4.42, dd, J=8.8Hz, 4.8Hz, 1H: 4.52, br-d, J=14.8Hz, 2H: 4.56, s, 1H: 4.71, s, 1H: 7.57, ddd, J=7.6Hz, 4.0Hz, 1.6Hz, 1H: 7.61, ddd, J=9.6Hz, 4.0Hz, 1.6Hz, 1H: 7.85, t, J=7.6Hz, 1H
$^{13}$C (100MHz:$CD_3OD$:25°C) 12.68, 24.05, 24.65, 25.20, 31.81, 34.02, 35.15, 47.33, 47.77, 52.31, 53.68, 55.98, 57.24, 59.68, 117.14, 117.34, 117.85, 117.98, 118.11, 118.23, 125.82, 125.95, 129.90, 130.07, 133.36, 135.11, 135.21, 135.72, 160.21, 162.71, 162.99, 163.44, 163.73, 167.10, 076.79, 176.98
MS: $[M+H]^+$ calculated: 507.244, found: 507.2
HRMS: $C_{25}H_{36}FN_4O_4S$ $[M+H]^+$ calculated: 507.2441, found: 507.2426

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 20.97 min was obtained.

[Example 17]

Ethyl N-(N-(4-(1-hexamethyleneiminoimidoyl)-2-fluorobenzoyl)-β-ethyl-α,α-dimethyl-β-alanyl)-4-piperidineacetate

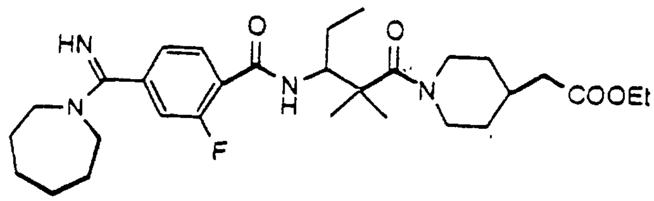

The same procedure as in Example 3 was performed with ethyl N-(N-(4-cyano-2-fluorobenzoyl)-β-ethyl-α,α-dimethyl-β-alanyl)-4-piperidineacetate (1.48 g, 3.3 mmol) using hexamethyleneimine (3.7 ml) as the amine component to give the titled compound (469 mg, 26%).
NMR: $^1$H (270MHz: $CDCl_3$: 25°C) 0.95, t, J=9.7Hz, 3H: 1.18, br-q, J=12.4Hz, 2H: 1.26, t, J=6.8Hz, 3H: 1.31, s, 3H: 1.36, s, 3H: 1.52-1.87, m, 10H: 1.87-2.16, m, 3H: 2.25, d, J=6.8Hz, 2H: 2.65-3.00, m, 2H: 3.41, br-t, J=5.4Hz, 2H: 3.74-3.86, m, 2H: 4.13, q, J=7.3Hz, 2H: 4.06-4.22, m, 1H: 4.42, br-d, J=13.2Hz, 2H: 7.20-7.37, m, 2H: 7.98, t, J=7.8Hz, 1H
$^{13}$C (67.5MHz:$CDCl_3$:25°C) 11.51, 14.20, 23.70, 23.84, 23.96, 25.17, 26.33, 27.40, 28.84, 32.00, 32.15, 33.13, 40.83, 45.04, 45.39, 46.56, 50.46, 51.88, 60.28, 60.36, 77.20, 115.61, 116.01, 123.45, 123.50, 125.82, 126.02, 132.56, 133.11,

133.23, 157.82, 160.55, 161.10, 161.54, 162.87, 163.13, 172.22, 174.75
MS: $[M+H]^+$ calculated: 545.350, found: 545.3
HRMS: $C_{30}H_{45}FN_4O_4$ $[M+H]^+$ calculated: 545.3502, found: 545.3512

[Example 18]

N-(N-(4-(1-hexamethyleneiminoimidoyl)-2-fluorobenzoyl)-β-ethyl-α,α-dimethyl-β-alanyl)-4-piperidineacetic acid

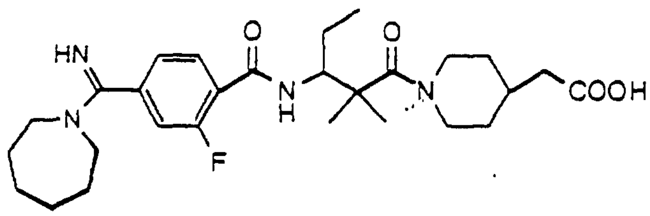

The same procedure as in Example 2 was performed with ethyl N-(N-(4-(1-hexamethyleneiminoimidoyl)-2-fluorobenzoyl) -β-ethyl-α,α-dimethyl-β-alanyl)-4-piperidineacetate (150 mg, 0.28 mmol) prepared in Example 17 to give the titled compound (103 mg, 72%).
NMR: $^1H$ (400MHz: $CD_3OD$: 25°C) 0.97, t, J=7.6Hz, 3H: 1.13-1.28, m, 2H: 1.26, s, 3H: 1.34, s, 3H: 1.48-1.60, m, 2H: 1.60, 1.72, m, 4H: 1.72-1.79, m, 2H: 1.85, br-t, J=11.6Hz, 2H: 1.91-2.01, m, 2H: 2.01-2.13, m, 1H: 2.26, d, J=7.6Hz, 2H: 2.74-3.09, m, 2H: 3.47, t, J=5.6Hz, 2H: 3.74, t, J=6.0Hz, 2H: 4.42, t, J=5.6Hz, 1H: 4.52, br-d, J=13.2Hz, 2H: 7.49, dd, J=8.0Hz, 1.6Hz, 1H: 7.55, dd, J=10.4Hz, 1.6Hz, 1H: 7.84, t, J=6.8Hz, 1H
$^{13}C$ (100MHz:$CD_3OD$:25°C) 12.68, 24.03, 24.65, 25.22, 27.14, 28.04, 29.28, 30.38, 34.04, 34.15, 35.15, 42.34, 47.35, 47.70, 52.05, 54.04, 59.62, 117.63, 117.81, 118.07, 125.79, 129.26, 129.41, 133.30, 135.52, 135.61, 165.60, 167.21, 176.81, 176.99
MS: $[M+H]^+$ calculated: 517.319, found: 517.4
HRMS: $C_{28}H_{42}FN_4O_4$ $[M+H]^+$ calculated: 517.3189, found: 517.3177

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 23.23 min was obtained.

[Example 19]

Ethyl N-(N-(4-(N-thiomorpholinoimidoyl)-2-fluorobenzoyl)-β-ethyl-α,α-dimethyl-β-alanyl)-4-piperidineacetate

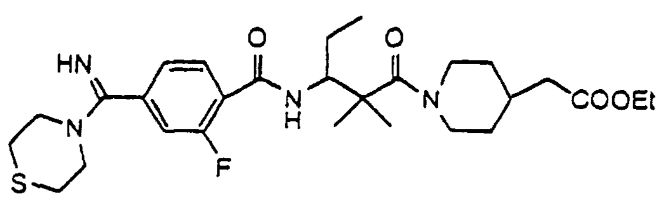

The same procedure as in Example 3 was performed with ethyl N-(N-(4-cyano-2-fluorobenzoyl)-β-ethyl-α,α-dimethyl-β-alanyl)-4-piperidineacetate (1.48 g, 3.3 mmol) using thiomorpholine (3.3 ml) as the amine component to give the titled compound (275 mg, 15%).
NMR: $^1H$ (270MHz: $CDCl_3$: 25°C) 0.94, t, J=7.6Hz, 3H: 1.18, br-q, J=13.2Hz, 2H: 1.26, t, J=6.8Hz, 3H: 1.31, s, 3H: 1.37, s, 3H: 1.55-1.89, m, 4H: 1.95-2.15, m, 1H: 2.25, d, J=6.8Hz, 2H: 2.58-2.70, m, 2H: 2.72-3.00, m, 4H: 3.60-3.73,

m, 2H: 3.85-4.26, m, 3H: 4.13, q, J=6.8Hz, 2H: 4.40, br-d, J=12.4hz, 2H: 7.25-7.39, m, 2H: 8.00, t, J=8.9Hz, 1H
$^{13}C$ (67.5MHz:$CDCl_3$:25°C) 11.54, 14.22, 23.84, 24.06, 26.68, 27.97, 31.97, 32.15, 33.12, 40.82, 45.04, 45.39, 46.47, 50.05, 52.83, 60.41, 60.68, 77.20, 114.07, 116.10, 116.50, 118.39, 123.85, 123.89, 126.39, 126.58, 132.34, 132.48, 132.74, 157.92, 160.80, 161.35, 161.64, 162.71, 163.16, 172.24, 174.81
MS: $[M+H]^+$ calculated: 549.291, found: 549.4
HRMS: $C_{29}H_{42}FN_4O_4S$ $[M+H]^+$ calculated: 549.2910, found: 549.2931

[Example 20]

N-(N-(4-(N-thiomorpholinoimidoyl)-2-fluorobenzoyl)-β-ethyl-α,α-dimethyl-β-alanyl)-4-piperidineacetic acid

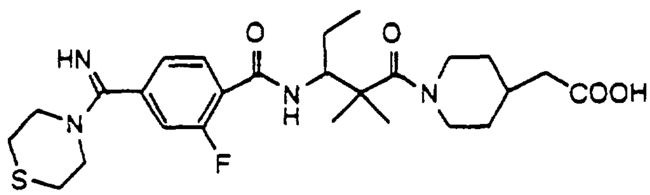

The same procedure as in Example 2 was performed with ethyl N-(N-(4-(N-thiomorpholinoimidoyl)-2-fluorobenzoyl)-β-ethyl-α,α-dimethyl-β-alanyl)-4-piperidineacetate (50 mg, 0.091 mmol) prepared in Example 19 to give the titled compound (40 mg, 84%).
NMR: $^1H$ (400MHz: $CD_3OD$: 25°C) 0.97, t, J=7.2Hz, 3H: 1.16-1.27, m, 2H: 1.26, s, 3H: 1.34, s, 3H: 1.53-1.61, m, 2H: 1.85, br-t, J=11.8Hz, 2H: 2.01-2.13, m, 1H: 2.26, d, J=7.2Hz, 2H: 2.71-2.74, m, 2H: 2.94-2.96, m, 2H: 2.80-3.02, m, 2H: 3.68-3.71, m, 2H: 4.05-4.08, m, 2H: 4.41, dd, J=4.8Hz, 8.8Hz, 2H: 4.52, br-d, J=13.2Hz, 2H: 7.53, dd, J=1.2Hz, 7.6Hz, 1H: 7.59, dd, J=1.6Hz, 10.4Hz, 1H: 7.85, t, J=7.2Hz, 1H
$^{13}C$ (100MHz:$CD_3OD$:25°C) 12.68, 24.05, 24.65, 25.22, 28.00, 29.28, 34.02, 24.13, 35.15, 42.32, 47.37, 47.70, 51.74, 55.09, 59.71, 118.23, 118.49, 126.71, 129.70, 129.85, 133.45, 134.84, 134.93, 160.35, 162.86, 165.49, 167.08, 176.79, 176.99
MS: $[M+H]^+$ calculated: 521.260, found: 521.3
HRMS: $C_{26}H_{38}FN_4O_4S$ $[M+H]^+$ calculated: 521.2597, found: 521.2598

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 21.49 min was obtained.

[Example 21]

Ethyl N-(N-(4-(4-methyl-1-piperazinoimidoyl)-2-fluorobenzoyl)-β-ethyl-α,α-dimethyl-β-alanyl)-4-piperidineacetate

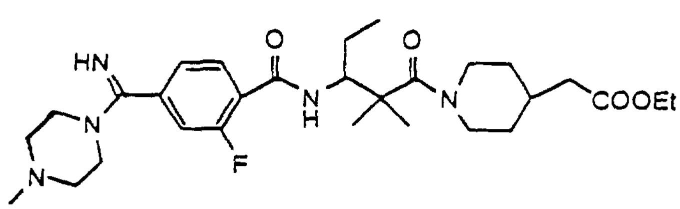

The same procedure as in Example 3 was performed with ethyl N-(N-(4-cyano-2-fluorobenzoyl)-β-ethyl-α,α-dimethyl-β-alanyl)-4-piperidineacetate (1.48 g, 3.3 mmol) using 1-methylpiperazine (3.7 ml) as the amine component

to give the titled compound (514 mg, 28%).
NMR: $^1$H (270MHz: $CDCl_3$: 25°C) 0.90, t, J=7.3Hz, 3H: 1.03-1.40, m, 2H: 1.25, t, J=7.3Hz, 3H: 1.28, s, 3H: 1.32, s, 3H: 1.48-1.92, m, 4H: 2.03, br-s, 1H: 2.23, d, J=6.5Hz, 2H: 2.65-3.00, m, 2H: 2.88, s, 3H: 3.42, br-s, 2H: 3.61, br-s, 2H: 3.78, br-s, 2H: 3.95-4.17, m, 1H: 4.11, q, J=6.8Hz, 2H: 4.19-4.50, m, 4H: 7.44, m, 2H: 7.86, m, 1H $^{13}C$ (67.5MHz: $CDCl_3$:25°C) 11.42, 14.15, 23.75, 23.92, 31.86, 32.02, 33.03, 40.76, 42.87, 43.83, 45.00, 45.48, 46.39, 46.82, 51.28, 51.87, 60.42, 60.70, 77.20, 113.90, 116.65, 117.03, 118.18, 124.38, 126.83, 127.03, 131.38, 131.50, 132.22, 157.66, 160.44, 160.98, 161.39, 161.51, 162.05, 162.98, 164.10, 172.27, 174.88
MS: $[M+H]^+$ calculated: 546.346, found: 546.4
HRMS: $C_{29}H_{45}FN_5O_4$ $[M+H]^+$ calculated: 546.3455, found: 546.3443

[Example 22]

N-(N-(4-(4-methyl-1-piperazinoimidoyl)-2-fluorobenzoyl)-β-ethyl-α,α-dimethyl-β-alanyl)-4-piperidineacetic acid

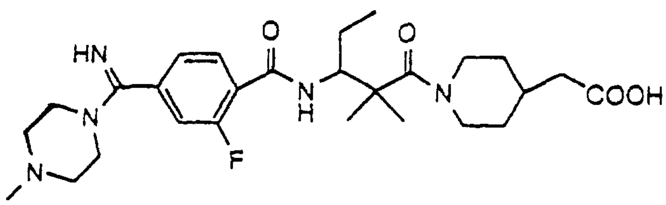

The same procedure as in Example 2 was performed with ethyl N-(N-(4-(4-methyl-1-piperazinoimidoyl)-2-fluorobenzoyl)-β-ethyl-α,α-dimethyl-β-alanyl)-4-piperidineacetate (60 mg, 0.11 mmol) prepared in Example 21 to give the titled compound (43 mg, 76%).
NMR: $^1$H (400MHZ: $CD_3OD$: 25°C) 0.97, t, J=7.6Hz, 3H: 1.14-1.27, m, 2H: 1.26, s, 3H: 1.34, s, 3H: 1.50-1.63, m, 2H: 1.84, br-t, J=10.0Hz, 2H: 2.01-2.13, m, 1H: 2.25, d, J=6.8Hz, 2H: 2.78-3.02, m, 2H: 2.93, s, 3H: 3.36, br-s, 2H: 3.54, br-s, 2H: 3.74, br-s, 2H: 4.09, br-s, 2H: 4.40, dd, J=4.4Hz, 10.0Hz, 1H: 4.51, br-d, J=13.6Hz, 2H: 7.56, dd, J=1.2Hz, 7.6Hz, 1H: 7.61, dd, J=1.2Hz, 10.0Hz, 1H: 7.88, t, J=7.2Hz, 1H
$^{13}C$ (100MHz:$CD_3OD$:25°C) 12.68, 24.12, 24.67, 25.18, 34.02, 34.11, 35.13, 42.32, 44.59, 45.87, 47.73, 53.53, 54.39, 59.87, 118.58, 118.86, 126.56, 130.34, 133.60, 133.94, 162.84, 163.79, 166.61, 166.90, 176.79, 176.99, 186.89,
MS: $[M+H]^+$ calculated: 518.314, found: 518.4
HRMS: $C_{27}H_{41}FN_5O_4$ $[M+H]^+$ calculated: 518.3142, found: 518.3125

HPLC analysis:

The compound obtained was subjected to analytical HPLC using a Wakosil-115C18HG column (φ4.6 x 250 mm) at a flow rate of 1.0 ml/min at room temperature with elution in a gradient of 10-55% acetonitrile in 0.1% TFA (30min). As a result, a spectrum having a single peak at a retention time of 16.69 min was obtained.

[Example 23]

Ethyl N-(N-(4-(4-phenyl-1-piperazinoimidoyl)-2-fluorobenzoyl)-β-ethyl-α,α-dimethyl-β-alanyl)-4-piperidineacetate

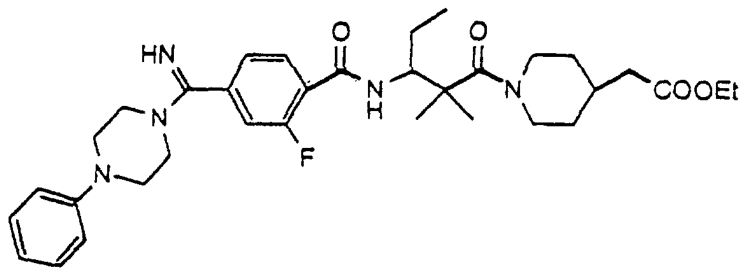

The same procedure as in Example 3 was performed with ethyl N-(N-(4-cyano-2-fluorobenzoyl)-β-ethyl-α,α-dimethyl-β-alanyl)-4-piperidineacetate (1.48 g, 3.3 mmol) using 1-phenylpiperazine (5.0 g) as the amine component to give the titled compound (237 mg, 12%).
NMR: $^{1}H$ (270MHz: $CDCl_3$: 25°C) 0.94, t, J=7.3Hz, 3H: 1.09-1.22, m, 2H: 1.26, t, J=7.0Hz, 3H: 1.31, s, 3H: 1.36, s, 3H: 1.57-1.22, m, 2H :1.79, br-d, J=11.3Hz, 2H: 1.95-2.12, m, 1H: 2.24, d, J=7.0Hz, 2H: 2.71-2.91, m, 2H: 3.21, br-s, 2H: 3.43, br-s, 2H: 3.57, br-s, 2H: 4.04-4.20, m, 3H: 4.13, q, J=7.1Hz, 2H: 4.40, br-d, J=13.5Hz, 2H: 6.90-6.98, m, 3H: 7.26-6.99, m, 4H: 8.00, t, J=7.3Hz, 1H
$^{13}C$ (67.5MHz:$CDCl_3$:25°C) 11.60, 14.30, 23.83, 24.00, 24.13, 31.99, 32.21, 33.14, 40.87, 45.29, 45.62, 46.56, 46.91, 49.00, 49.81, 50.02, 60.57, 61.19, 116.42, 116.80, 117.41, 122.54, 124.24, 126.62, 126.82, 129.66, 132.12, 132.25, 132.84, 148.84, 157.99, 160.42, 160.97, 161.72, 162.94, 163.11, 172.37, 175.17
MS: $[M+H]^+$ calculated: 608.361, found: 608.4

[Example 24]

N-(N-(4-(4-phenyl-1-piperazinoimidoyl)-2-fluorobenzoyl)-β-ethyl-α,α-dimethyl-β-alanyl)-4-piperidineacetic acid

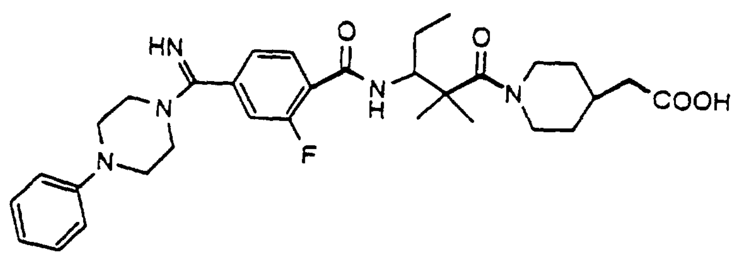

The same procedure as in Example 2 was performed with ethyl N-(N-(4-(4-phenyl-1-piperazinoimidoyl)-2-fluorobenzoyl)-β-ethyl-α,α-dimethyl-β-alanyl)-4-piperidineacetate (50 mg, 0.082 mmol) prepared in Example 23 to give the titled compound (41 mg, 86%).
NMR: $^{1}H$ (400MHz: $CD_3OD$: 25°C) 0.98, t, J=6.8Hz, 3H: 1.14-1.31, m, 2H: 1.27, s, 3H: 1.35, s, 3H: 1.52-1.64, m, 2H: 1.79-1.92, m, 2H: 2.06, br-s, 1H: 2.26, d, J=6.4Hz, 2H: 2.60-3.10, m, 2H: 3.48, br-s, 2H: 3.62, br-s, 2H: 3.96, br-s, 2H: 4.37-4.46, m, 1H: 4.52, br-d, J=12.8Hz, 2H: 6.89, t, J=7.2Hz, 1H: 6.99, d, J=7.6Hz, 2H: 7.27, t, J=7.6Hz, 2H: 7.56, d, J=7.6Hz, 1H: 7.62, d, J=9.6Hz, 1H: 7.87, t, J=7.6Hz, 1H
$^{13}C$ (100MHz:$CD_3OD$:25°C) 12.68, 24.18, 24.73, 25.28, 34.05, 34.13, 35.15, 42.34, 47.33, 47.73, 51.89, 60.02, 111.32, 118.49, 118.62, 118.75, 122.79, 126.46, 129.76, 131.09, 133.47, 134.73, 134.82, 152.38, 157.85, 160.39, 162.89, 165.42, 166.97, 176.77, 177.07
MS: $[M+H]^+$ calculated: 580.330, found: 580.3
HRMS: $C_{32}H_{43}FN_5O_4$ $[M+H]^+$ calculated: 580.3298, found: 580.3310

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 26.52 min was obtained.

[Example 25]

Ethyl N-(N-(4-(4-(4-fluorophenyl)-1-piperazinoimidoyl)-2-fluorobenzoyl)-β-ethyl-α,α-dimethyl-β-alanyl)-4-piperidineacetate

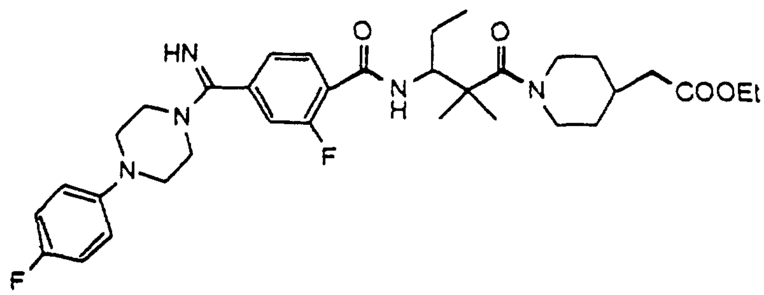

The same procedure as in Example 3 was performed with ethyl N-(N-(4-cyano-2-fluorobenzoyl)-β-ethyl-α,α-dimethyl-β-alanyl)-4-piperidineacetate (1.48 g, 3.3 mmol) using 1-(4-fluorophenyl)piperazine (5.9 g) as the amine component to give the titled compound (396 mg, 19%).
NMR: $^1$H (400MHz: $CDCl_3$: 25°C) 0.92, t, J=7.2Hz, 3H: 1.16, br-q, J=12.0Hz, 2H: 1.24, t, J=7.0Hz, 3H: 1.30, s, 3H: 1.35, s, 3H: 1.60-1.72, m, 2H: 1.78, br-d, 11.6Hz, 2H: 1.97-2.08, m, 1H: 2.23, d, J=6.8Hz, 2H: 2.82, br-s, 2H: 3.15, m, 2H: 3.37, m, 2H: 3.59, m, 2H: 4.02-4.14, m, 3H: 4.11, q, J=7.2Hz, 2H: 4.35, br-d, J=12.4Hz, 2H: 6.92, dd, J=4.4Hz, 9.6Hz, 2H: 6.99, t, J=8.4Hz, 2H: 7.33, dd, J=10.8Hz, 14.0Hz, 2H: 7.96, t, J=7.2Hz, 1H
$^{13}$C (67.5MHz:$CDCl_3$:25°C) 11.60, 14.28, 23.83, 24.00, 24.14, 31.99, 32.21, 33.14, 40.86, 45.20, 45.56, 46.53, 46.98, 49.81, 49.96, 50.80, 60.57, 61.19, 115.99, 116.32, 116.42, 116.81, 119.36, 119.49, 124.25, 126.59, 126.79, 132.14, 132.26, 132.80, 145.74, 145.77, 156.79, 157.96, 160.35, 160.97, 161.69, 162.94, 163.11, 172.36, 175.14
MS: $[M+H]^+$ calculated: 626.352, found: 626.4

[Example 26]

N-(N-(4-(4-(4-fluorophenyl)-1-piperazinoimidoyl)-2-fluorobenzoyl)-β-ethyl-α,α-dimethyl-β-alanyl)-4-piperidineacetic acid

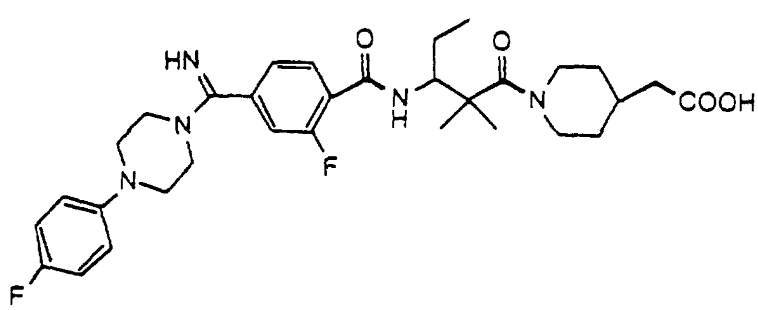

The same procedure as in Example 2 was performed with ethyl N-(N-(4-(4-(4-fluorophenyl)-1-piperazinoimidoyl)-2-fluorobenzoyl)-β-ethyl-α,α-dimethyl-β-alanyl)-4-piperidineacetate (80 mg, 0.13 mmol) prepared in Example 25 to give the titled compound (55 mg, 72%).
NMR: $^1$H (400MHz: $CD_3OD$: 25°C) 0.98, t, J=7.6Hz, 3H: 1.15-1.28, m, 2H: 1.26, s, 3H: 1.35, s, 3H: 1.51-1.62, m, 2H:

1.84, br-t, J=11.2Hz, 2H: 1.98-2.15, m, 1H: 2.25, d, J=7.2Hz, 2H: 2.75-3.07, m, 2H: 3.20, t, J=4.8Hz, 2H: 3.40, t, J=4.8Hz, 2H: 3.61, t, J=4.4Hz, 2H: 3.95, t, J=4.8Hz, 2H: 4.41, dd, J=5.2Hz, 9.6Hz, 1H: 4.52, br-d, J=13.2Hz, 2H: 7.01, d, J=6.8Hz, 4H: 7.56, dd, J=1.2Hz, 7.6Hz, 1H: 7.61, d, J=10.4Hz, 1H: 7.87, t, J=6.8Hz, 1H
$^{13}C$ (100MHz:$CD_3OD$:25°C) 12.68, 24.09, 24.67, 25.20, 34.04, 34.13, 35.15, 42.32, 47.31, 47.73, 50.86, 51.89, 51.96, 59.77, 117.27, 117.48, 118.49, 118.75, 120.67, 120.76, 126.46, 129.77, 129.92, 133.41, 134.71, 134.80, 149.14, 165.40, 167.06, 176.79, 176.98
MS: $[M+H]^+$ calculated: 598.320, found: 598.1

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 27.32 min was obtained.

[Example 27]

Benzyl N-(N-(4-(4-morpholinoimidoyl)benzoyl-β-phenyl-α,α-dimethyl-β-alanyl)-4-piperidineacetate

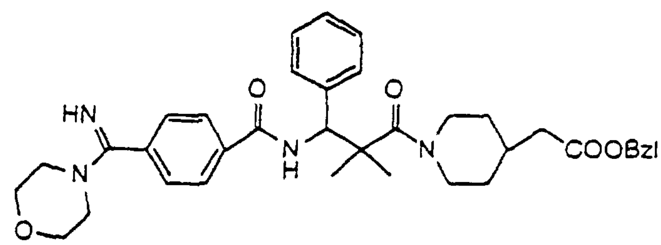

The same procedure as in Example 1 was performed with benzyl N-(N-β-ethyl-α,α-dimethyl-β-alanyl)-4-piperidineacetate (0.5 g, 0.98 mmol) which had been prepared by condensation of N-BOC-β-ethyl-α,α-dimethyl-β-alanine with benzyl 4-piperidineacetate in the same manner as in Example 1 to give the titled compound (270 mg, 44%) in a powder form.
NMR: $^1H$ (270MHz: $CDCl_3$: 25°C) 0.73-1.38, m, 2H: 1.30, s, 3H: s, 3H: 1.45, s, 3H: 1.63-1.82, m, 2H: 1.92-2.12, m, 1H: 2.25, d, J=7.6Hz, 2H: 2.32-2.88, m, 2H: 3.28-3.42, m, 2H: 3.58-3.73, m, 2H: 3.81-4.05, m, 4H: 4.16-4.45, m, 2H: 5.05-5.16, m, 1H: 5.10, s, 2H: 7.20-7.49, m, 12H: 7.79-7.91, m, 2H:
$^{13}C$ (67.5MHz:$CDCl_3$:25°C) 24.93, 26.21, 29.68, 31.74, 32.04, 32.09, 32.95, 32.99, 40.62, 45.42, 45.65, 46.93, 47.42, 50.14, 62.84, 65.74, 66.30, 77.20, 127.10, 127.67, 128.22, 128.31, 128.57, 128.95, 129.76, 130.83, 135.77, 135.93, 138.49, 139.19, 161.57, 164.42, 164.83, 171.93, 175.42
MS: $[M+H]^+$ calculated: 625.338, found: 625.4

[Example 28]

N-(N-(4-(4-morpholinoimidoyl)benzoyl-β-phenyl-α,α-dimethyl-β-alanyl)-4-piperidineacetic acid

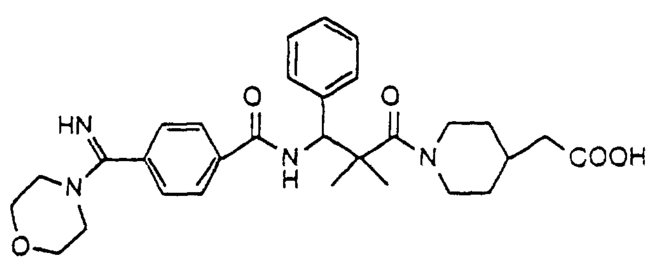

Benzyl N-(N-(4-(4-morpholinoimidoyl)benzoyl-β-phenyl-α,α-dimethyl-β-alanyl)-4-piperidineacetate (54 mg, 0.086 mmol) prepared in Example 27 was dissolved in a 80% aqueous methanol solution containing 2% acetic acid (10 ml). To the resultant solution was added 5% palladium carbon (10 mg) and the mixture was stirred overnight in a hydrogen atmosphere. After distilling off the solvent from the mixture solution, the residue was dissolved in a 1N aqueous acetic acid solution and then purified by HPLC in the same manner as in Example 2 to give the titled compound (32 mg, 69%).
NMR: $^1$H (400MHz: $CD_3OD$: 25°C) 1.07, dq, J=1.4Hz, 9.2Hz, 1H: 1.22, dq, J=1.3Hz, 9.2Hz, 1H: 1.30, s, 3H: 1.35, s, 3H: 1.74-1.85, m, 2H: 1.93-2.08, m, 1H: 2.13-2.24, m, 2H: 2.80-2.97, m, 2H: 3.44, t, J=4.4Hz, 2H: 3.73, t, J=4.8Hz, 2H: 3.80, t, J=3.6Hz, 2H: 3.92, dd, J=3.6Hz, 5.6Hz, 2H: 4.50, br-t, J=9.2Hz, 2H: 5.57, s, 1H: 7.26-7.35, m, 4H: 7.43, br-d, J=8.8Hz, 1H: 7.72, d, J=8.0Hz, 2H: 7.98, dt, J=8.4Hz, 2.0Hz, 2H
$^{13}$C (100MHz:$CD_3OD$:25°C) 25.29, 25.99, 33.89, 33.94, 35.02, 42.28, 47.61, 47.75, 47.92, 48.67, 52.18, 61.98, 67.10, 68.11, 129.57, 129.96, 130.36, 130.35, 133.65, 140.56, 140.66, 144.75, 163.30, 164.47, 166.92, 169.00, 176.79, 176.92
MS: $[M+H]^+$ calculated: 535.292, found: 535.3
HRMS $C_{30}H_{39}N_4O_5$ $[M+H]^+$ calculated: 535.2920, found: 535.2935

HPLC analysis:

The compound obtained was subjected to analytical HPLC using a Wakosil-115C18HG column (ϕ4.6 x 250 mm) at a flow rate of 1.0 ml/min at room temperature with elution in a gradient of 10-55% acetonitrile in 0.1% TFA (30min). As a result, a spectrum having a single peak at a retention time of 23.61 min was obtained.

[Example 29]

Methyl N-(N-(4-(1-pyrrolidinoimidoyl)-2-fluorobenzoyl)-β-ethyl-α,α-dimethyl-β-alanyl)-4-piperidineacetate

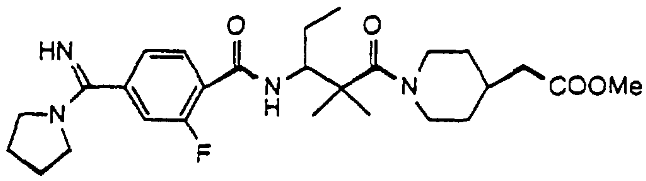

N-BOC-β-ethyl-α,α-dimethyl-β-alanine was condensed with methyl 4-piperidineacetate in the same manner as in Example 1 to give methyl N-(N-β-ethyl-α,α-dimethyl-β-alanyl)-4-piperidineacetate. This compound was used to give methyl N-(N-(4-cyano-2-fluorobenzoyl)-β-ethyl-α,α-dimethyl-β-alanyl)-4-piperidineacetate in the same manner as in the first step of Example 3. Subsequently, the same procedure as in the second step of Example 3 was performed with the compound thus obtained (1.0 g, 2.3 mmol) using pyrrolidine (1.9 ml) as the amine component to give the titled compound (248 mg, 21%).
NMR: $^1$H (400MHz: $CDCl_3$: 25°C) 0.92, t, J=7.4Hz, 3H: 1.15, q, J=12.6Hz, 2H: 1.29, s, 3H: 1.34, s, 3H: 1.161-1.69, m, 2H: 1.77, br-d, J=12Hz/ 2H: 1.96, br-t, J=6.6Hz, 2H: 1.98-2.07, m, 1H: 2.13, br-t, J=6.4Hz, 2H: 2.24, d, J=7.2Hz, 2H: 2.81, br-s, 2H: 3.41, t, J=6.6Hz, 2H: 3.65, s, 3H: 3.73, m, 2H: 4.08, br-t, J=8.8Hz, 1H: 4.38, br-d, J=12.8Hz, 2H: 7.31, br-d, J=10.8Hz, 1H: 7.36, br-d, J=8.0Hz, 1H: 7.98, t, J=7.6Hz, 1H
$^{13}$C (100MHz:$CDCl_3$:25°C) 11.65, 23.96, 24.05, 24.16, 24.96, 25.60, 32.11, 32.29, 33.23, 40.67, 45.19, 45.50, 46.67, 49.28, 51.59, 52.27, 60.81, 115.87, 116.13, 123.66, 123.70, 126.26, 126.39, 132.77, 133.35, 133.45, 158.59, 161.10, 162.85, 172.73, 175.00
MS: $[M+H]^+$ calculated: 503.303, found: 503.4

[Comparative Example 1]

Ethyl N-(N-4-amidino-2-fluorobenzoyl-β-ethyl-α,α-dimethyl-β-alanyl)-4-piperidineacetate

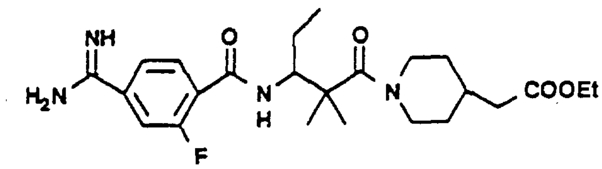

The same procedure as in Example 3 was performed with ethyl N-(N-(4-cyano-2-fluorobenzoyl)-β-ethyl-α,α-dimethyl-β-alanyl)-4-piperidineacetate (0.42 mg, 0.94 mmol) using ammonium acetate (500 mg) as the amine component to give the titled compound (200 mg, 48%).
MS: $[M+H]^+$ calculated 463.272, found 463.4

[Experimental Example 1]

Platelet Aggregation-Inhibiting Ability of the Compounds of the Present Invention

(Measurement of in vitro Human Platelet Aggregation using PRP)

Healthy male volunteers who had not taken any medicines for at least two weeks were selected as subjects. Blood was collected from the forearm vein of each subject on an empty stomach using a #19 needle and a plastic syringe preliminarily charged with 1/10 volume of a 3.8% sodium citrate solution. Immediately after the blood collection, the syringe was shaken gently to mix the blood with the sodium citrate solution. The mixed blood was centrifuged (1100 rpm, 250 g) at room temperature for 15 minutes and the rotation was stopped without applying the brake. Then, the supernatant was collected with a graduated pipette to obtain platelet-rich plasma (PRP). The PRP was stored at room temperature. The blood remaining after the centrifugation was further centrifuged (3500 rpm, 1500 g) at room temperature for 15 minutes and the rotation was stopped without applying the brake. The supernatant was collected to obtain platelet-poor plasma (PPP). After the preparation of the PRP, the number of platelets was counted and only samples containing $2 \times 10^8$ or more of platelets per milliliter were subjected to the following experiments.

Platelet aggregation was measured using an 8-channel aggregometer (Hematracer, Nikoh Bioscience, Tokyo, Japan) on the basis of the change in light transmittance through the PRP as follows.

First, the PPP and PRP (each 200 µl) were placed in glass cuvettes and incubated at 37°C. Thereafter, the transmittance was measured. The transmittance of the PPP was defined as 100% and that of the PRP as 0%.

Then, 10 µl of physiological saline or a sample-containing physiological saline was added to the PRP and incubated at 37°C for one minute. A collagen solution (10 µl) at a concentration of 100 µg/ml was added to the PRP-containing solution (final concentration: 5 µg/ml) to induce aggregation and thereafter the transmittance was measured over 7 minutes. After the aggregation with collagen and ADP was confirmed, only those samples in which the maximum percent aggregation with collagen was at least 70% were subjected to the experiment.

The sample was dissolved in physiological saline to give a concentration of $2.2 \times 10^{-2}$ M and a 2-fold dilution series was prepared for use in the experiments. The samples insoluble in the physiological saline were dissolved in physiological saline containing 10% DMSO (dimethyl sulfoxide).

The results were calculated as follows:

$$\text{Percent Aggregation inhibition} = \frac{\text{Maximum percent aggregation upon the addition of sample}}{\text{Maximum percent aggregation upon the addtion of physiological saline}}$$

A graph was constructed by plotting the percent of aggregation inhibition against the sample concentration and the concentration at which the aggregation was inhibited by 50% ($IC_{50}$) was calculated from the graph. $IC_{50}$ of each sample is shown in Table 1.

Table 1.

| Platelet Aggregation-Inhibiting Activity of the Compounds of the Present Invention | |
|---|---|
| Compound | Activity ($IC_{50}$, μM) |
| The Compound Prepared in Ex.2 | 0.22 |
| The Compound Prepared in Ex.4 | 0.15 |
| The Compound Prepared in Ex.8 | 0.22 |
| The Compound Prepared in Ex.10 | 0.087 |
| The Compound Prepared in Ex.12 | 0.12 |
| The Compound Prepared in Ex.14 | 0.052 |
| The Compound Prepared in Ex.16 | 0.043 |
| The Compound Prepared in Ex.18 | 0.23 |
| The Compound Prepared in Ex.20 | 0.12 |
| The Compound Prepared in Ex.22 | 0.069 |
| The Compound Prepared in Ex.24 | 0.057 |
| The Compound Prepared in Ex.26 | 0.087 |
| The Compound Prepared in Ex.28 | 0.24 |

Table 1 shows that the compounds of the present invention have a high platelet-aggregation inhibiting activity.
The compounds prepared in Examples 1, 3, 5, 7, 9, 11, 13, 15, 17, 19, 21, 23, 25, 27 and 29 are prodrugs.

[Experimental Example 2]

Time Course of the Concentration in Plasma after Oral Administration (Oral Administration Test)

Male SD rats which had been starved from the previous day were used in this experiment. Two test substances, the compounds of Example 4 and Comparative Example 1, were dissolved in distilled water and administered into the stomach of rats with an oral probe. Each compound was administered to each rat in an amount of 10 mg per kg of body weight. Blood samples were collected 30, 60 and 120 minutes after the administration. The concentration of each compound which was in an active state (i.e., free carboxylic acid) in plasma was determined by HPLC.
The results are shown in Figure 1. In this Figure, the horizontal axis represents the time after the oral administration and the vertical axis represents the concentration of the active form of each compound in plasma. Each point represents the mean value for three rats and the standard error. The compound of Example 4 within the scope of the invention revealed a significantly higher concentration in plasma than the compound of Comparative Example 1 at every point of determination. When the peak values at 30 minutes after the oral administration are compared, the difference was about 10 times as great. This shows that the compound of Example 4 (i.e. a compound of the invention) clearly has a higher bioavailability than the compound of Comparative Example 1.

[Experimental Example 3] Acute Toxicity Test

The compounds of the present invention were intravenously injected into mice in an amount of 1 g/kg but no toxicity was observed.

[Formulation Example 1]

Each of the compounds prepared in Examples 1-29 (100 mg) was dissolved in 100 ml of physiological saline. Under aseptic conditions, the obtained solution was charged in a 2.5 ml ampule and the ampule was sealed to prepare an injection preparation.

[Formulation Example 2]

A mixture (1 ml) of ethanol and water was added to a mixture consisting of each of the compounds prepared in Examples 1-29 (500 mg), crystalline cellulose (50 mg) and lactose (450 mg) and the two mixtures were blended intimately. The blend was granulated by a conventional method to prepare granules.

INDUSTRIAL APPLICABILITY

According to the present invention, are provided compounds which antagonize fibrinogen receptors to thereby exhibit a high platelet aggregation-inhibiting activity, and which are excellent in in vivo stability against the cleavage with proteolytic enzymes and bioavailability. These compounds are useful as agents for inhibiting platelet aggregation. The pharmaceutical preparations comprising the compounds as active ingredient are very effective in preventing platelet thrombosis, thromboembolism and reocclusion during and after thrombolytic therapy, preventing platelet thrombosis, thromboembolism and reocclusion after angioplasty of coronary and other arteries and after coronary artery bypass procedures, preventing unstable angina and myocardial infraction, improving peripheral circulatory bloodstream and inhibiting blood coagulation during extracorporeal circulation.

**Claims**

1. A compound of the following general formula (I) and a pharmaceutically acceptable salt thereof:

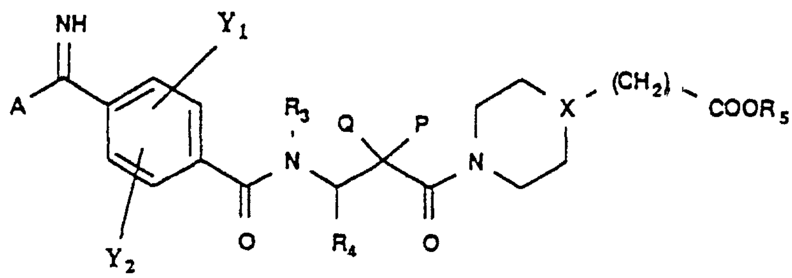

(I)

wherein A is a tertiary amine represented by the following general formula (II), (III) or (IV):

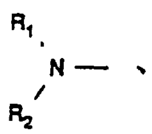

(II)

,

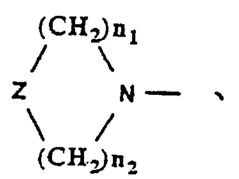

(III)

, or

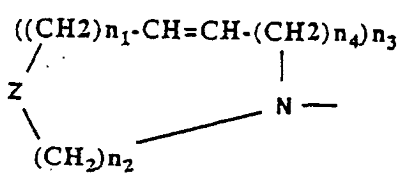

(IV)

wherein $R_1$ and $R_2$ are each independently a $C_1$-$C_{10}$ alkyl, $C_2$-$C_{10}$ alkenyl, $C_2$-$C_{10}$ alkynyl, ar($C_1$-$C_3$)alkyl or aryl; Z is oxygen, sulfur,

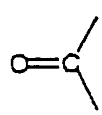

,

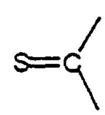

;

carbon having hydrogen, a $C_1$-$C_{10}$ alkyl, $C_2$-$C_{10}$ alkanyl, $C_2$-$C_{10}$ alkynyl, ar($C_1$-$C_3$)alkyl, hydroxyl or substituted hydroxyl, amino or substituted amino, nitro, nitrooxy, nitrosooxy, halogen, thiol or substituted thiol, or aryl as its side chain; or nitrogen having hydrogen, a $C_1$-$C_{10}$ alkyl, $C_2$-$C_{10}$ alkenyl, $C_2$-$C_{10}$ alkynyl, ar($C_1$-$C_3$)alkyl, hydroxyl or substituted hydroxyl, amino or substituted amino, nitro, nitroso or aryl as its side chain;
and wherein the substituent of the "substituted hydroxyl" when Z is carbon or nitrogen is benzyl, phenethyl, trityl, methyl, ethyl, propyl, isopropyl, tert-butyl, acetyl, benzoyl, nitro, nitroso, tetrahydropyranyl or methoxymethyl;
the substituent of the "substituted amino" when Z is carbon or nitrogen is benzyl, phenethyl, trityl, methyl, ethyl, propyl, isopropyl, tert-butyl, acetyl, benzoyl, benzyloxycarbonyl, methoxycarbonyl, or nitroso;
the substituent of the "substituted thiol" when Z is carbon is benzyl, phenethyl, trityl, methyl, ethyl, propyl, isopropyl,

tert-butyl, or nitroso;
$n_1$, $n_2$ and $n_4$ are each independently an integer of 0 to 6 and $n_3$ is an integer of 0 to 3;
wherein the term "aryl" for $R_1$ and $R_2$ is selected from phenyl; the aromatic heterocycles pyridyl, furyl, thiophene, oxazolyl and thiazolyl; and the condensed polycyclic hydrocarbons naphthyl and anthranyl, which all may be substituted with at least one of the substituents consisting of a $C_1$-$C_3$ alkyl, hydroxy $C_1$-$C_3$ alkyl, hydroxyl and protected hydroxyl;
$R_3$ is hydrogen, a $C_1$-$C_{10}$ alkyl, $C_2$-$C_{10}$ alkenyl, $C_2$-$C_{10}$ alkynyl, ar($C_1$-$C_{10}$)alkyl or aryl;
$R_4$ is hydrogen, a $C_1$-$C_{10}$ alkyl, $C_2$-$C_{10}$ alkenyl, $C_2$-$C_{10}$ alkynyl, hydroxy($C_1$-$C_{10}$)alkyl, nitrooxy($C_1$-$C_{10}$)alkyl, nitrosooxy($C_1$-$C_{10}$)alkyl, amino($C_1$-$C_{10}$)alkyl or heterocycle-substituted $C_1$-$C_{10}$ alkyl, an ar($C_1$-$C_{10}$)alkyl, ar($C_2$-$C_{10}$) alkenyl or ar($C_2$-$C_{10}$)alkynyl the aryl moiety of which may have a $C_1$-$C_{10}$ alkyl, halogen, nitro, amino, carboxyl, hydroxy($C_1$-$C_{10}$)alkyl, hydroxyl or protected hydroxyl; an aryl or heterocyclic group which may have a $C_1$-$C_{10}$ alkyl, halogen, nitro, amino, carboxyl, hydroxy($C_1$-$C_{10}$)alkyl, hydroxyl or protected hydroxyl; a cycloalkyl with a 3-8 membered ring the ring moiety of which may have a $C_1$-$C_{10}$ alkyl, halogen, nitro, amino, carboxyl, hydroxy($C_1$-$C_{10}$)alkyl, hydroxyl or protected hydroxyl, or a $C_1$-$C_{10}$ alkyl, a $C_2$-$C_{10}$ alkynyl or a $C_2$-$C_{10}$ alkenyl which are substituted with the cycloalkyl; or a $C_1$-$C_{10}$ alkyloxy;
P and Q are each independently a $C_1$-$C_{10}$ alkyl, or when combined together, form a cycloalkyl with the adjacent carbon atom;
$R_5$ is hydrogen or a physiologically cleavable carboxyl-protecting group selected from alkoxy groups capable of forming esters, amino acid residues having a free amino group, protected amino acid residues thereof and 1-acyloxyalkoxides;
$Y_1$ and $Y_2$ are each independently hydrogen, a $C_1$-$C_{10}$ alkyl, halogen, hydroxy, a $C_1$-$C_{10}$ alkoxy, a $C_1$-$C_{10}$ acyloxy, an acyl, carboxyl, a $C_1$-$C_{10}$ alkoxycarbonyl, nitro or trifluoromethyl;
and wherein the "aryl" for $R_3$ is selected from phenyl and the condensed polycyclic hydrocarbons naphthyl and anthranyl, wherein the aromatic ring may be substituted with at least one of the substituents consisting of a lower alkyl, hydroxy($C_1$-$C_{10}$)alkyl, hydroxyl and protected hydroxyl;
the "heterocycle-substituted lower alkyl" for $R_4$ is a $C_1$-$C_{10}$ alkyl substituted with a 5-6 membered heterocycle or unsaturated condensed heterocycle containing at least one hetero atom;
the "aryl" for $R_4$ is phenyl, naphthyl or anthranyl, wherein the aromatic ring may be substituted with a $C_1$-$C_3$ alkyl, halogen, nitro, amino, carboxyl, hydroxy($C_1$-$C_3$)alkyl, hydroxyl or protected hydroxyl;
and the "heterocycle" for $R_4$ is a pyridyl, furyl, pyrrolyl, thiophene, oxazolyl, thiazolyl, imidazolyl, pyrazolyl, tetrazolyl, triazolyl, indolyl, benzothiazolyl, benzimidazolyl, benzoxazolyl, coumaryl, carbazolyl, pyranyl, pyronyl, quinolyl, isoquinolyl, pyrimidyl, pyrazinyl, piperidyl, piperazyl or tetrahydrofuryl group, wherein the heterocycle may be substituted with a $C_1$-$C_3$ alkyl, halogen, nitro, amino, carboxyl, hydroxy($C_1$-$C_3$)alkyl, hydroxyl or protected hydroxyl.

**2.** The compound and its pharmaceutically acceptable salt of claim 1, wherein A is a cyclic tertiary amine.

**3.** The compound and its pharmaceutically acceptable salt of any one of claims 1 and 2, wherein P and Q are both methyl.

**4.** A pharmaceutical preparation comprising the compound or its pharmaceutically acceptable salt of any one of claims 1 to 3 as an active ingredient.

**5.** The pharmaceutical preparation of claim 4 which is used for inhibiting platelet aggregation.

**6.** The pharmaceutical preparation of claim 4 which is used for inhibiting blood coagulation during extracorporeal circulation.

**7.** The pharmaceutical preparation of claim 4 which is used for inhibiting the reocclusion of coronary arteries.

## Patentansprüche

**1.** Verbindung mit der folgenden allgemeinen Formel (I) und ein pharmazeutisch verträgliches Salz davon:

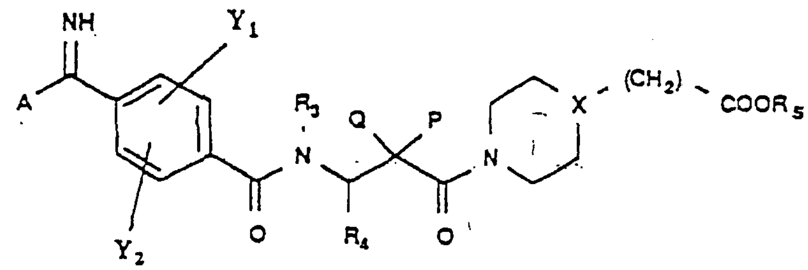

(I)

wobei A ein tertiäres Amin ist, dargestellt durch die folgende allgemeine Formel (II), (III) oder (IV):

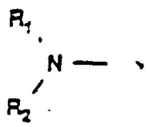

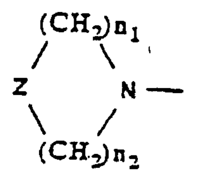

oder

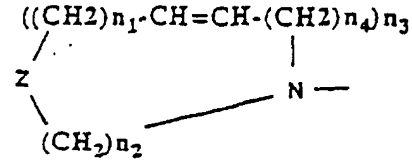

(II) (III) (IV)

wobei $R_1$ und $R_2$ unabhängig voneinander einen $C_1$-$C_{10}$-Alkyl-, $C_2$-$C_{10}$-Alkenyl-, $C_2$-$C_{10}$-Alkinyl-, ar($C_1$-$C_3$)Alkyl- oder einen Arylrest bedeuten;
Z ein Sauerstoffatom, Schwefelatom,

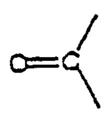

,

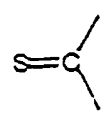

;

ein Kohlenstoffatom mit einem Wasserstoffatom, einem $C_1$-$C_{10}$-Alkyl-, $C_2$-$C_{10}$-Alkenyl-, $C_2$-$C_{10}$-Alkinyl-, ar($C_1$-$C_3$) Alkyl-, Hydroxyl- oder substituierten Hydroxyl-, Amino- oder substituierten Amino-, Nitro-, Nitrooxy-, Nitrosooxy-, Halogen-, Thiol- oder substituierten Thiol- oder Arylrest als Seitenkette; oder ein Stickstoffatom mit einem Wasserstoffatom, einem $C_1$-$C_{10}$-Alkyl-, $C_2$-$C_{10}$-Alkenyl-, $C_2$-$C_{10}$-Alkinyl-, ar($C_1$-$C_3$)Alkyl-, Hydroxyl- oder substituierten Hydroxyl-, Amino- oder substituierten Amino-, Nitro-, Nitroso- oder Arylrest als Seitenkette ist;
und wobei der Substituent der "substituierten Hydroxylgruppe" eine Benzyl-, Phenethyl-, Trityl-, Methyl-, Ethyl-, Propyl-, Isopropyl-, tert-Butyl-, Acetyl-, Benzoyl-, Nitro-, Nitroso-, Tetrahydropyranyl oder Methoxymethylgruppe ist, wenn Z ein Kohlenstoff- oder Stickstoffatom ist;
der Substituent der "substituierten Aminogruppe" eine Benzyl-, Phenethyl-, Trityl-, Methyl-, Ethyl-, Propyl-, Isopropyl-, tert-Butyl-, Acetyl-, Benzoyl-, Benzyloxycarbonyl, Methoxycarbonyl oder Nitrosogruppe ist, wenn Z ein Kohlenstoff- oder Stickstoffatom ist;
der Substituent der "substituierten Thiolgruppe" eine Benzyl-, Phenethyl-, Trityl-, Methyl-, Ethyl-, Propyl-, Isopropyl, tert-Butyl, oder Nitrosogruppe ist, wenn Z ein Kohlenstoffatom ist;
$n_1$, $n_2$ und $n_4$ unabhängig voneinander eine ganze Zahl von 0 bis 6 sind und $n_3$ eine ganze Zahl von 0 bis 3 ist;
wobei der Begriff "Arylrest" für $R_1$ und $R_2$ ausgewählt ist aus einer Phenylgruppe; den aromatischen Heterocyclen Pyridyl-, Furyl-, Thiophen-, Oxazolyl und Thiazolylgruppe, und den kondensierten, polycyclischen Kohlenwasserstoffen Naphthyl- und Anthranylgruppe, die alle mit mindestens einem der Substituenten bestehend aus einem $C_1$-$C_3$-Alkyl-, Hydroxy-$C_1$-$C_3$-Alkyl-, Hydroxyl- und geschütztem Hydroxylrest substituiert sein können;
$R_3$ ein Wasserstoffatom, ein $C_1$-$C_{10}$-Alkyl-, $C_2$-$C_{10}$-Alkenyl-, $C_2$-$C_{10}$-Alkinyl-, ar($C_1$-$C_{10}$)Alkyl- oder Arylrest ist;
$R_4$ ein Wasserstoffatom, ein $C_1$-$C_{10}$-Alkyl-, $C_2$-$C_{10}$-Alkenyl-, $C_2$-$C_{10}$-Alkinyl-, Hydroxy($C_1$-$C_{10}$)alkyl-, Nitrooxy ($C_1$-$C_{10}$)alkyl-, Nitrosooxy($C_1$-$C_{10}$)alkyl-, Amino($C_1$-$C_{10}$)alkyl- oder Heterocyclus-substituierter $C_1$-$C_{10}$-Alkyl-, ein ar($C_1$-$C_{10}$)Alkyl-, ar($C_2$-$C_{10}$)Alkenyl- oder ar($C_2$-$C_{10}$)Alkinylrest deren, Arylkomponente einen $C_1$-$C_{10}$-Alkyl-, Ha-

logen-, Nitro-, Amino-, Carboxyl-, Hydroxy($C_1$-$C_{10}$)Alkyl-, Hydroxyl- oder geschützten Hydroxylrest haben kann; ein Aryl- oder ein heterocyclischer Rest, der einen $C_1$-$C_{10}$-Alkyl-, Halogen-, Nitro-, Amino-, Carboxyl-, Hydroxy ($C_1$-$C_{10}$)alkyl-, Hydroxyl- oder geschützten Hydroxylrest haben kann; ein Cycloalkylrest mit einem 3- bis 8-gliedrigen Ring, dessen Ringanteil einen ($C_1$-$C_{10}$)Alkyl-, Halogen-, Nitro-, Amino-, Carboxyl-, Hydroxy($C_1$-$C_{10}$)alkyl-, Hydroxyl- oder geschützten Hydroxylrest haben kann, oder ein $C_1$-$C_{10}$-Alkyl-, $C_2$-$C_{10}$-Alkinyl- oder ein $C_2$-$C_{10}$-Alkenylrest, die mit dem Cycloalkylrest substituiert sind; oder ein $C_1$-$C_{10}$-Alkyloxyrest ist;
P und Q unabhängig voneinander ein $C_1$-$C_{10}$-Alkylrest sind, oder zusammengenommen einen Cycloalkylrest mit dem benachbarten Kohlenstoffatom bilden;
$R_5$ ein Wasserstoffatom oder eine physiologisch spaltbare CarboxylSchutzgruppe ist, ausgewählt aus Alkoxyresten, die Ester bilden können, Aminosäureresten mit einer freien, Aminogruppe, geschützten Aminosäureresten davon und 1-Acyloxyalkoxiden;
$Y_1$ und $Y_2$ unabhängig voneinander ein Wasserstoffatom, einen $C_1$-$C_{10}$-Alkyl-, Halogen-, Hydroxy-, einen $C_1$-$C_{10}$-Alkoxy-, einen $C_1$-$C_{10}$-Acyloxy-, einen Acyl-, einen Carboxyl-, einen $C_1$-$C_{10}$-Alkoxycarbonyl-, Nitro-, oder Trifluoromethylrest bedeuten;
und wobei der "Arylrest" für $R_3$ ausgewählt ist aus einer Phenylgruppe und den kondensierten polycyclischen Kohlenwasserstoffen Naphthyl- und Anthranylgruppe, wobei der aromatische Ring mit mindestens einem der Substituenten bestehend aus einem Niederalkyl-, Hydroxy($C_1$-$C_{10}$)alkyl-, Hydroxyl- oder geschütztem Hydroxylrest substituiert sein kann;
der "Heterocyclus-substituierte Niederalkylrest" für $R_4$ ein $C_1$-$C_{10}$-Alkylrest ist, substituiert mit einem 5- bis 6-gliedrigen Heterocyclus oder ungesättigten kondensierten Heterocyclus, der mindestens ein Heteroatom enthält;
der "Arylrest" für $R_4$ eine Phenyl-, Naphthyl- oder Anthranylgruppe ist, wobei der aromatische Ring mit einem $C_1$-$C_3$-Alkyl-, Halogen-, Nitro-, Amino-, Carboxyl-, Hydroxy($C_1$-$C_3$)alkyl-, Hydroxyl- oder geschützem Hydroxylrest substituiert sein kann;
und der "Heterocyclus" für $R_4$ eine Pyridyl-, Furyl-, Pyrrolyl-, Thiophen-, Oxazolyl-, Thiazolyl-, Imidazolyl-, Pyrazolyl-, Tetrazolyl-, Triazolyl-, Indolyl-, Benzothiazolyl-, Benzimidazolyl-, Benzoxazolyl-, Cumaryl-, Carbazolyl-, Pyranyl-, Pyronyl-, Chinolyl-, Isochinolyl-, Pyrimidyl-, Pyrazinyl-, Piperidyl-, Piperazyl- oder Tetrahydrofurylgruppe ist, wobei der Heterocyclus mit einem $C_1$-$C_3$-Alkyl-, Halogen-, Nitro-, Amino-, Carboxyl-, Hydroxy($C_1$-$C_3$)alkyl-, Hydroxyl- oder geschütztem Hydroxylrest substituiert sein kann.

**2.** Verbindung und ihr pharmazeutisch verträgliches Salz nach Anspruch 1, wobei A ein cyclisches tertiäres Amin ist.

**3.** Verbindung und ihr pharmazeutisch verträgliches Salz nach einem der Ansprüche 1 und 2, wobei P und Q beide Methylgruppen sind.

**4.** Arzneimittel umfassend die Verbindung oder ihr pharmazeutisch verträgliches Salz nach einem der Ansprüche 1 bis 3 als Wirkstoff.

**5.** Arzneimittel nach Anspruch 4, das zur Hemmung der Blutplättchenaggregation verwendet wird.

**6.** Arzneimittel nach Anspruch 4, das zur Hemmung der Blutgerinnung während extrakorporaler Zirkulation verwendet wird.

**7.** Arzneimittel nach Anspruch 4, welches zur Hemmung der Reokklusion von Koronararterien verwendet wird.

## Revendications

**1.** Composé de la formule générale suivante (I) et un de ses sels pharmaceutiquement acceptables:

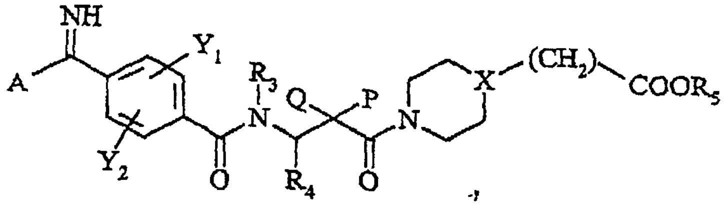

(I)

dans laquelle A est une amine tertiaire représentée par la formule générale suivante (II), (III) ou (IV):

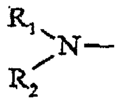 , 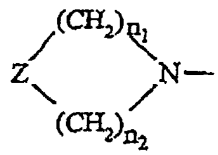 ou 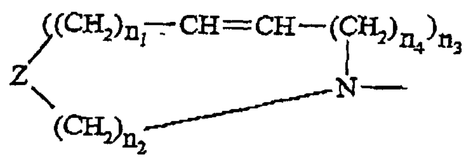

(II) (III) (IV)

dans lesquelles $R_1$ et $R_2$ sont chacun indépendamment un groupe alkyle en $C_1$-$C_{10}$, alcényle en $C_2$-$C_{10}$, alcynyle en $C_2$-$C_{10}$, ar(alkyle en $C_1$-$C_3$) ou aryle;
Z est un atome d'oxygène, de soufre, un groupe

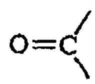 , 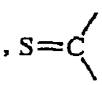 ;

un atome de carbone portant des atomes d'hydrogène, un groupe alkyle en $C_1$-$C_{10}$, alcényle en $C_2$-$C_{10}$, alcynyle en $C_2$-$C_{10}$, ar(alkyle en $C_1$-$C_3$), hydroxy ou hydroxy substitué, amino ou amino substitué, nitro, nitrooxy, nitrosoxy, un atome d'halogène, un groupe thiol ou thiol substitué ou aryle en tant que chaîne latérale; ou un atome d'azote portant un atome d'hydrogène, un groupe alkyle en $C_1$-$C_{10}$, alcényle en $C_2$-$C_{10}$, alcynyle en $C_2$-$C_{10}$, ar(alkyle en $C_1$-$C_3$), hydroxy ou hydroxy substitué, amino ou amino substitué, nitro, nitroso ou aryle en tant que chaîne latérale;
et dans lesquelles le substituant du groupe "hydroxy substitué" lorsque Z est un atome de carbone ou d'azote est un groupe benzyle, phénéthyle, trityle, méthyle, éthyle, propyle, isopropyle, tert-butyle, acétyle, benzoyle, nitro, nitroso, tétrahydropyranyle ou méthoxyméthyle;
le substituant du groupe "amino substitué" lorsque Z est un atome de carbone ou d'azote est un groupe benzyle, phénéthyle, trityle, méthyle, éthyle, propyle, isopropyle, tert-butyle, acétyle, benzoyle, benzyloxycarbonyle, méthoxycarbonyle ou nitroso;
le substituant du groupe "thiol substitué" lorsque Z est un atome de carbone est un groupe benzyle, phénéthyle, trityle, méthyle, éthyle, propyle, isopropyle, tert-butyle ou nitroso;
$n_1$, $n_2$ et $n_4$ sont chacun indépendamment un entier de 0 à 6 et $n_3$ est un entier de 0 à 3; dans lesquelles le terme "aryle" pour $R_1$ et $R_2$ est choisi parmi un groupe phényle; les hétérocycles aromatiques pyridyle, finyle, thiophène, oxazolyle et thiazolyle; et les hydrocarbures polycycliques condensés naphtyle et anthranyle, qui peuvent tous être substitués par au moins un des substituants constitués par un groupe alkyle en $C_1$-$C_3$, hydroxy(alkyle en $C_1$-$C_3$), hydroxy et hydroxy protégé;
$R_3$ est un atome d'hydrogène, un groupe alkyle en $C_1$-$C_{10}$, alcényle en $C_2$-$C_{10}$, alcynyle en $C_2$-$C_{10}$, ar(alkyle en $C_1$-$C_{10}$) ou aryle;
$R_4$ est un atome d'hydrogène, un groupe alkyle en $C_1$-$C_{10}$, alcényle en $C_2$-$C_{10}$, alcynyle en $C_2$-$C_{10}$, hydroxy(alkyle en $C_1$-$C_{10}$), nitrooxy(alkyle en $C_1$-$C_{10}$), nitrosooxy(alkyle en $C_1$-$C_{10}$), ammo(alkyle en $C_1$-$C_{10}$) ou (hétérocycle substitué)(alkyle en $C_1$-$C_{10}$), ar(alkyle en $C_1$-$C_{10}$), ar(alcényle en $C_2$-$C_{10}$) ou ar(alcynyle en $C_2$-$C_{10}$), dont le reste aryle peut comporter un groupe alkyle en $C_1$-$C_{10}$, un atome d'halogène, un groupe nitro, amino, carboxyle, hydroxy (alkyle en $C_1$-$C_{10}$), hydroxy ou hydroxy protégé; un groupe aryle ou hétérocyclique qui peut comporter un groupe alkyle en $C_1$-$C_{10}$, un atome d'halogène, un groupe nitro, amino, carboxyle, hydroxy(alkyle en $C_1$-$C_{10}$), hydroxy ou hydroxy protégé; un groupe cycloalkyle avec un cycle à 3 à 8 chaînons, dont le noyau cyclique peut comporter un groupe alkyle en $C_1$-$C_{10}$, un atome d'halogène, un groupe nitro, amino, carboxyle, hydroxy(alkyle en $C_1$-$C_{10}$), hydroxy ou hydroxy protégé, ou un groupe alkyle en $C_1$-$C_{10}$, alcényle en $C_2$-$C_{10}$ ou alcynyle en $C_2$-$C_{10}$, qui sont substitués par un groupe cycloalkyle; ou un groupe alcoxy en $C_1$-$C_{10}$;
P et Q sont chacun indépendamment un groupe alkyle en $C_1$-$C_{10}$ ou, lorsqu'ils sont combinés ensemble, ils forment un groupe cycloalkyle avec l'atome de carbone adjacent;
$R_5$ est un atome d'hydrogène ou un groupe protecteur d'un groupe carboxyle physiologiquement clivable choisi parmi les groupes alcoxy capables de former des esters, des résidus acide aminé comportant un groupe amino libre, leurs résidus acide aminé protégés et les 1-acyloxyalcoolates;
$Y_1$ et $Y_2$ sont chacun indépendamment un atome d'hydrogène, un groupe alkyle en $C_1$-$C_{10}$, un atome d'halogène, un groupe hydroxy, alcoxy en $C_1$-$C_{10}$, acyloxy en $C_1$-$C_{10}$, acyle, carboxyle, (alcoxy en $C_1$-$C_{10}$)carbonyle, nitro ou trifluorométhyle;
et dans lesquelles le terme "aryle" pour $R_3$ est choisi parmi un groupe phényle et les hydrocarbures polycycliques condensés naphtyle et anthranyle, où le cycle aromatique peut être substitué par au moins un des substituants

constitués par un groupe alkyle inférieur, hydroxy(alkyle en $C_1$-$C_{10}$), hydroxy et hydroxy protégé;
le terme "alkyle inférieur substitué par un hétérocycle" pour $R_4$ est un groupe alkyle en $C_1$-$C_{10}$ substitué par un hétérocycle à 5-6 chaînons ou un hétérocycle condensé insaturé contenant au moins un hétéroatome;
le terme "aryle" pour $R_4$ est un groupe phényle, naphtyle ou anthranyle, où le cycle aromatique peut être substitué par un groupe alkyle en $C_1$-$C_3$, un atome d'halogène, un groupe nitro, amino, carboxyle, hydroxy(alkyle en $C_1$-$C_3$), hydroxy ou hydroxy protégé;
et le terme "hétérocycle" pour $R_4$ est un groupe pyridyle, furyle, pyrrolyle, thiophène, oxazolyle, thiazolyle, imidazolyle, pyrazolyle, tétrazolyle, triazolyle, indolyle, benzothiazolyle, benzimidazolyle, benzoxazolyle, coumaryle, carbazolyle, pyranyle, pyronyle, quinolyle, isoquinolyle, pyrimidyle, pyrazinyle, pipéridyle, pipérazyle ou tétrahydrofuryle, où l'hétérocycle peut être substitué par un groupe alkyle en $C_1$-$C_3$, un atome d'halogène, un groupe nitro, amino, carboxyle, hydroxy(alkyle en $C_1$-$C_3$), hydroxy ou hydroxy protégé.

**2.** Composé et son sel pharmaceutiquement acceptable selon la revendication 1, dans lequel A est une amine tertiaire cyclique.

**3.** Composé et son sel pharmaceutiquement acceptable selon l'une quelconque des revendications 1 et 2, dans lequel P et Q sont tous deux un groupe méthyle.

**4.** Préparation pharmaceutique comprenant le composé ou son sel pharmaceutiquement acceptable selon l'une quelconque des revendications 1 à 3 en tant qu'ingrédient actif.

**5.** Préparation pharmaceutique selon la revendication 4, qui est utilisée pour inhiber l'agrégation plaquettaire.

**6.** Préparation pharmaceutique selon la revendication 4, qui est utilisée pour inhiber la coagulation du sang lors d'une circulation extracorporelle.

**7.** Préparation pharmaceutique selon la revendication 4, qui est utilisée pour inhiber la réocclusion des artères coronaires.

FIG.1

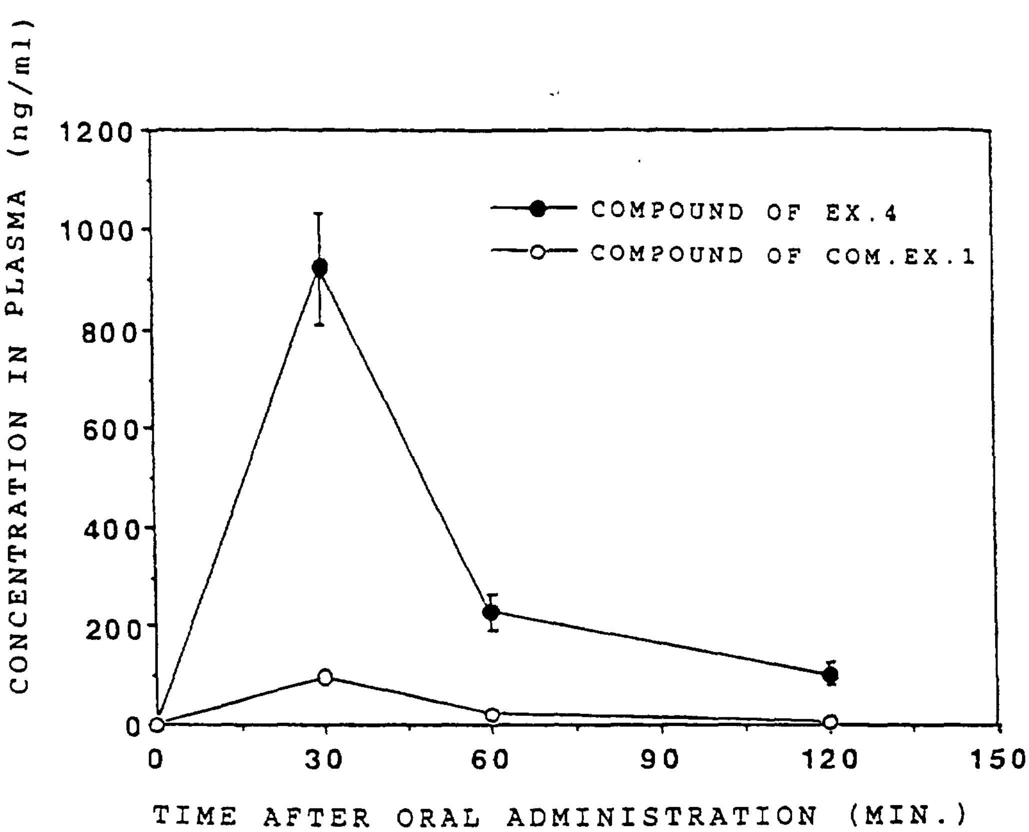


VARIATION OF CONCENTRATION IN PLASMA AFTER ORAL ADMINISTRATION